(19) **Europäisches Patentamt / European Patent Office / Office européen des brevets**

(11) **EP 4 565 075 B1**

(12) **EUROPEAN PATENT SPECIFICATION**

(45) Date of publication and mention
of the grant of the patent:
**03.06.2026 Bulletin 2026/23**

(21) Application number: **23726597.0**

(22) Date of filing: **21.04.2023**

(51) International Patent Classification (IPC):
*A23L 33/105* (2016.01)     *A23L 33/12* (2016.01)
*A61K 31/164* (2006.01)     *A61K 36/11* (2006.01)

(52) Cooperative Patent Classification (CPC):
(C-Sets available)
**A23L 33/105; A23L 33/12; A61K 9/0056;
A61K 9/143; A61K 31/137; A61K 36/11**     (Cont.)

(86) International application number:
**PCT/IB2023/054089**

(87) International publication number:
**WO 2024/028659 (08.02.2024 Gazette 2024/06)**

(54) **COMBINATION OF PALMITOYLETHANOLAMIDE AND EQUISETUM AND FOOD, NUTRACEUTICAL AND/OR PHARMACEUTICAL COMPOSITIONS CONTAINING IT FOR USE IN NEURONAL PROTECTION AND REGENERATION**

PALMITOYLETHANOLAMID- UND EQUISETUM- UND LEBENSMITTEL-, NUTRAZEUTISCHE UND/ODER PHARMAZEUTISCHE ZUSAMMENSETZUNGEN ZUR VERWENDUNG IM NEURONALEN SCHUTZ UND ZUR REGENERATION

COMBINAISON DE PALMITOYLÉTHANOLAMIDE ET D'EQUISETUM ET COMPOSITIONS ALIMENTAIRES, NUTRACEUTIQUES ET/OU PHARMACEUTIQUES LA CONTENANT POUR UNE UTILISATION DANS LA PROTECTION ET LA RÉGÉNÉRATION NEURONALES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **02.08.2022 IT 202200016404**

(43) Date of publication of application:
**11.06.2025 Bulletin 2025/24**

(73) Proprietor: **Agave S.r.l.**
**59100 Prato (IT)**

(72) Inventors:
• **UBERTI, Francesca**
**21012 Cassano Magnago (VA) (IT)**
• **TISO, Domenico**
**21012 Cassano Magnago (VA) (IT)**

(74) Representative: **Modiano, Micaela Nadia et al**
**Modiano & Partners**
**Via Meravigli, 16**
**20123 Milano (IT)**

(56) References cited:
• **CLAYTON PAUL ET AL: "Palmitoylethanolamide: A Natural Compound for Health Management",** INTERNATIONAL JOURNAL OF MOLECULAR SCIENCES, vol. 22, no. 10, 18 May 2021 (2021-05-18), pages 5305, XP093025568, DOI: 10.3390/ijms22105305
• **BATIR-MARIN DENISA ET AL: "Neuroprotective and Antioxidant Enhancing Properties of Selective Equisetum Extracts",** MOLECULES, vol. 26, no. 9, 28 April 2021 (2021-04-28), pages 2565, XP093025566, DOI: 10.3390/molecules26092565

(52) Cooperative Patent Classification (CPC): (Cont.)

C-Sets
**A61K 31/137, A61K 2300/00**

**Description**

FIELD OF INVENTION

It is an object of he present invention a synergistic combination of palmitoylethanolamide (in short PEA) and *Equisetum* and the food, nutraceutical and/or pharmaceutical compositions containing it, for use in a method for the prevention and repair of peripheral nervous system damage, and for use in a method for peripheral nervous system neuronal protection and regeneration.

TECHNICAL FIELD

The nervous system (NS) is a collection of organs, tissues and nerve cells capable of receiving, analyzing and processing stimuli from inside and outside the body. The nervous system generates responses appropriate to the contingent situation to promote survival.

The peripheral nervous system (PNS) is the "arm" of the central nervous system and transmits to the central nervous system (CNS) all the information data picked up inside and outside the organism. In addition, the PNS disseminates to the periphery all processing with origin in the CNS. Without the PNS, the CNS could not function properly.

The central nervous system (CNS) is the most important part of the nervous system, a true data processing and control center. It analyzes incoming information from the body's external and internal environment and formulates the most appropriate responses.

Neuronal damage may be due to nerve compression; two parameters must be taken into account when evaluating the effects of nerve compression: the extent of compression and the duration of compression. In experimental studies, it has been shown that even for minor compression, damage to the intraneural microcirculation is created and axonal transport is impaired, both rapid anterograde (for cell membrane constituents) and slow anterograde (for cytoskeleton constituents). Such blockage of anterograde axonal flow interferes with the replenishment of absolemma constituents and neurotransmitter precursors necessary for synaptic function.

The nerve ischemia that peripheral nerves undergo results in oxidative stress with a blocked supply of local energy metabolites, a peripheral increase in lipid hydroperoxides, and a decrease in antioxidant defense capabilities.

Peripheral nerve injuries are accompanied by inflammatory reactions that hinder the recovery of function. The reaction begins with inflammasome activation in motor neurons, which triggers a complex inflammatory reaction and microglia activation. In a physiological situation, the interaction between small fiber nociceptive endings and tissue mast cells conditions the peripheral pain threshold. Microglia control the nerve signal between first neuron and second-order neuron and is in turn regulated by palmitoylethanolamide (PEA).

In a pathological situation, the hyper-reactive mast cell induces peripheral sensitization (lowering of the algogenic threshold) and neurogenic inflammation (district neuroinflammation). During injury or dysfunction of the nervous system--peripheral, spinal, or supraspinal--microglia become activated and proliferate, triggering spinal neuroinflammation and neuropathic pain.

The manifestation of pain occurs as a result of even multiple qualified agonist stimuli, neurogenic stimuli (nerve signal intensity), immunogenic stimuli (cytokines, NFG, BDNF, NT3), mechanical stimuli (trauma), microbial stimuli (Gram$^+$/Gram$^-$) and central stimuli. These stimuli transform the phenotype and release of cytokines such as IL-1, IL-6, TNF-a, BDNF, resulting in neuronal damage, neuroinflammation, and chronic/neuropathic pain.

Palmitoylethanolamide (PEA) belongs to the N-acylethanolamine (NAE) family of biologically active endogenous lipids, which also includes the endogenous cannabinoid receptor ligand (anandamide) and satiety factor (oleoylethanolamide), and can potentially be used in a wide range of therapeutic areas, including pain and neurodegeneration, while at the same time being essentially free of undesirable effects in humans.

PEA is present in many plant and animal food sources and is also an endogenous substance synthesized when needed from membrane phospholipids; peripherally, PEA can modulate mast cell activation and reduce glial activation.

PEA exerts anti-inflammatory, analgesic, immunomodulatory, and neuroprotective effects and acts by restoring homeostasis. PEA's ability to modulate protective responses during inflammation stems from its membership in the homeostatic system that controls inflammation and can counteract the release of inflammatory mediators synthesized *de novo* by activated mast cells. PEA acts through a pleiotropic receptor-like mechanism in a receptor complex in which both membrane receptors (GPR55, CB2) and nuclear receptors (PPAR-a) participate.

Because of its poor bioavailability, PEA is generally administered in micronized or ultra-micronized form.

*Equisetum Arvense* is a well-known medicinal plant rich in phytochemical compounds such as flavonoids, phenolic acids, alkaloids, phytosterols, tannins, and triterpenoids and has antioxidant and anti-inflammatory activities. In addition, acting on oxidative stress prevents cell death, which is the goal of neuroprotection to minimize neural dysfunction through mechanisms to maintain the integrity of cellular interactions.

The antioxidant and anti-inflammatory action of *Equisetum Arvense* thus derives from its multiple effects, including stimulation of ATP biosynthesis and free radical scavenger systems, prevention as well as reduction of apoptosis by inhibiting oxidative stress, regulation of redox and glucose balance through SIRTs (1 and 2) and action in transactivation of "osmoprotective genes" (taurine transporter, aldose reductase, betaine/GABA and myo-inositol).

To appropriately counteract an inflammatory process affecting a nerve, it is necessary to act through a dual therapeutic approach: a symptomatic treatment - with nonsteroidal anti-inflammatory drugs, corticosteroids, muscle relaxants, and analgesics - is combined with a neurotrophic treatment, to promote the restoration of proper nerve cell function.

Therefore, it is understood that there is a need to find new therapeutic solutions that are able to address and solve the various problems associated with inflammatory processes and peripheral nervous system damage.

## Purposes of the invention

It is a first purpose of the invention to promote neuronal health through a combination of active ingredients that can act through different mechanisms of action.

It is another purpose of the invention a food, nutraceutical and/or pharmaceutical composition comprising the combination of active ingredients of the invention useful for exerting neuroprotection and neuronal regeneration activity.

It is a further purpose of the invention novel food, nutraceutical, and/or pharmaceutical formulations that allow optimal bioavailability of the active ingredients of the invention's combination and consequent real efficacy.

## Brief description of the Drawings

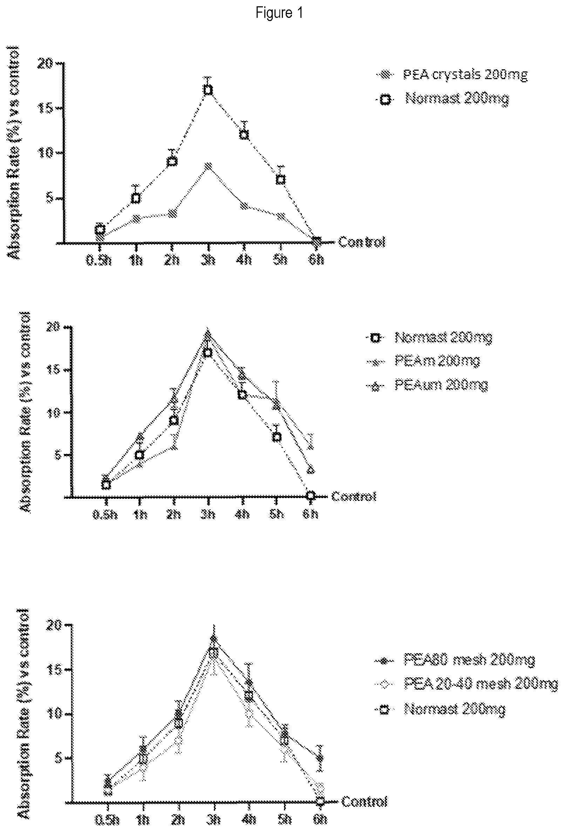

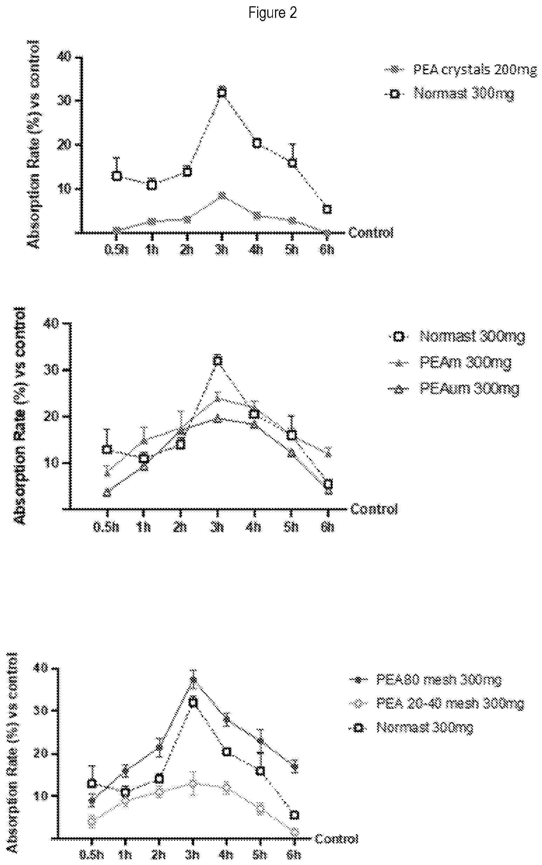

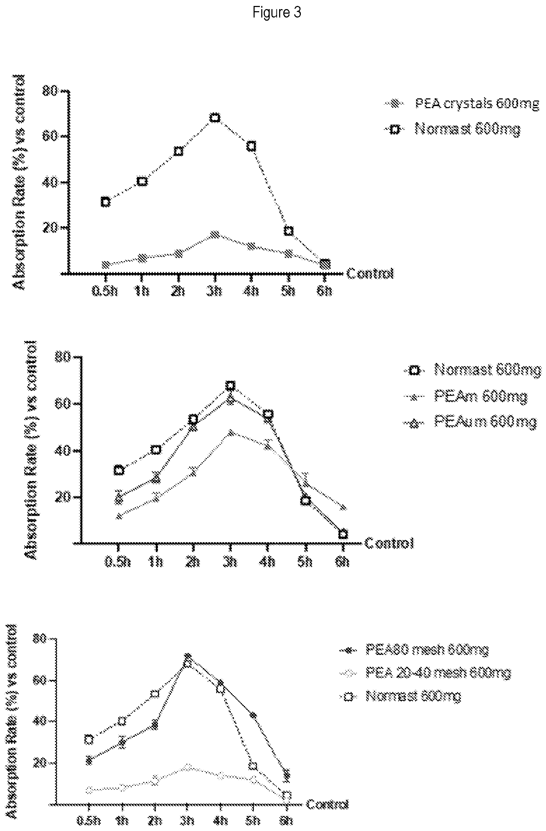

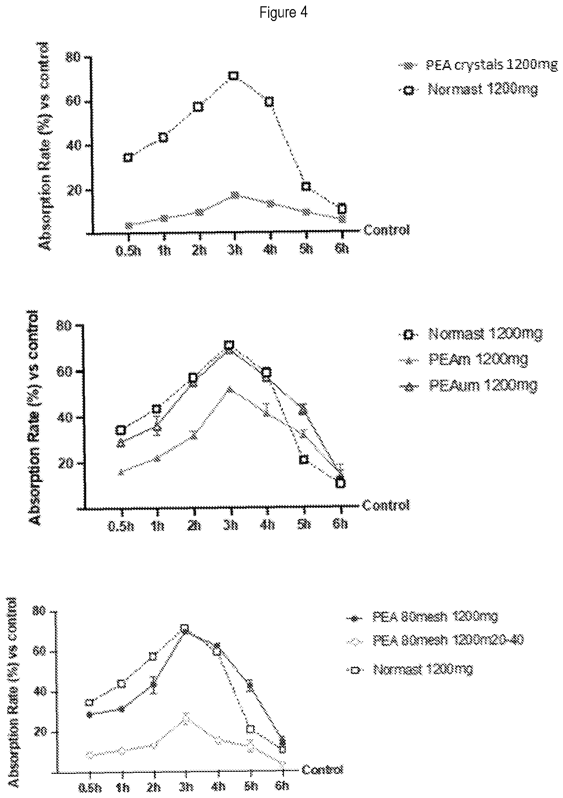

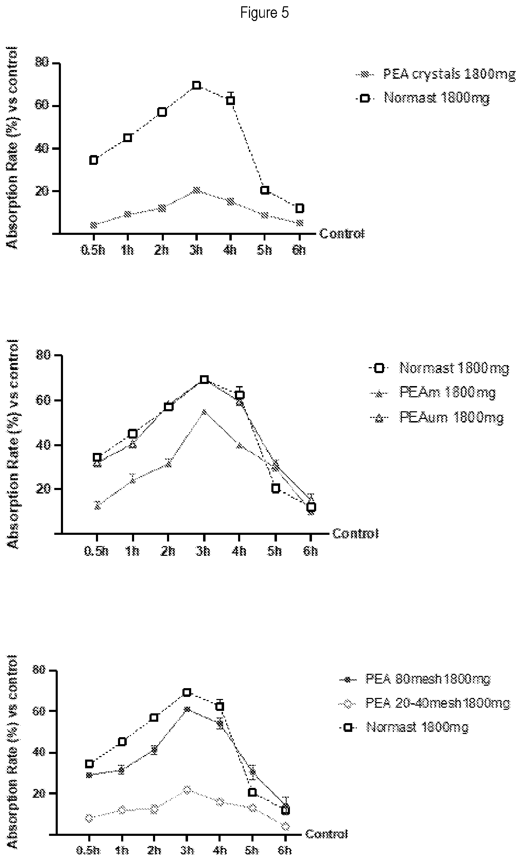

Figures 1 to 5 show PEA uptake compared with the commercially available product called Normast®.

Figures 6 and 7 show the absorption of PEA 80 mesh, *Equisetum* 10%, PEA 80 mesh+ *Equisetum* 10%, compared with Normast®.

Figure 8 shows Zo-1 analysis confirming the active role of TJs in the uptake of individual components and combination of the invention.

Figure 9 shows claudin analysis that confirms the active role of TJs in the uptake of compounds.

Figure 10 shows occludin analysis confirming the active role of TJs in the uptake of compounds.

Figure 11 shows the motor neuron/Schwann cell (MN-SC) model.

Figure 12 shows iotoneurons and Schwann cells.

Figure 13 shows the assessment of cell viability on the PNS 3D model to exclude possible toxicity.

Figure 14 shows the assessment of ROS production in terms of inhibition for the reduction of oxidative stress related to nerve damage.

Figure 15 shows the assessment of TNF-a (15a) and IL-2 production to establish anti-inflammatory capacity (15b).

Figure 16 shows the assessment of the activity of NGR1: Neuregulin 1 (16a) and MPZ: myelin zero protein (16b) to establish myelination and compact myelin formation.

Figure 17 shows the assessment of the activity of p75: regulator of myelin (17a) and ERB3: mediator of Schwann cell biological activity (17b) to determine the effectiveness for neuronal protection and regeneration in the peripheral nervous system.

Figure 18 shows the assessment of endocannabinoid receptor activity:CB1 (18a) and CB2 (18b).

Figure 19 shows the assessment of G-protein-coupled receptor 55 (GPR55) activity.

Figure 20 shows the assessment of GABAergic activity (20a) and production of NGF: neuronal growth factor (20b).

**Description of the invention**

The Applicant, after extensive and intensive research and development activity, has surprisingly found that a combination of palmitoylethanolamide and *Equisetum* can exert effective protective and regenerative effects at the neuronal level, particularly on the peripheral nervous system.

Thus, according to one of its aspects, it is an object of the invention a food, nutraceutical and/or pharmaceutical combination consisting of palmitoylethanolamide and *Equisetum* for use thereof in the protection and neuronal regeneration of the peripheral nervous system.

Palmitoylethanolamide or N-(2-hydroxyethyl)hexadecanamide (here also just "PEA"), is the naturally occurring ethanolamide derivative of hexadecanoic acid and is found in particularly abundant amounts in some foods, including egg yolk and soybeans. At room temperature and pressure it occurs as a solid. PEA is commercially available in various solid forms, particle sizes, or average particle sizes, and can be, for example, in crystalline, micronized, ultramicronized, 20, 40, 60, 80, 100, or 120 mesh, form. According to a preferred embodiment, PEA is PEA of about 80 mesh. Meshes are Anglo-Saxon units corresponding to the number of meshes per linear inch, generally used to determine particle size distribution of granular materials. For example, 80 mesh (0.0070 inches) corresponds to 0.177 mm or 177 microns.

*Equisetum* is a plant belonging to the family *Equisetaceae* and is commonly known as "horsetails." According to the present invention, the term "*Equisetum*" includes *Equisetum Arvense*. According to a preferred embodiment, the term "*Equisetum* " here means *Equisetum Arvense*.

In the combination of the invention, *Equisetum* may be used in any of its forms, for example, in powder form, as a fluid extract, mother tincture, or macerate; preferably the *Equisetum* used in the combination of the invention is in powder form and has a silica titer greater than 1%, more preferably greater than 5%, even more preferably greater than 7%, and with a maximum titer at 20%, preferably less than 15%, for example, a silica titer around 8%, or 10%, or 12%, preferably around 10%.

PEA and *Equisetum* are also referred to here generically as the "active ingredients" or "components" of the combination of the invention.

The phrase "neuronal protection and regeneration of the peripheral nervous system" here indicates the activity exerted by the combination of the invention that is capable of promoting neuronal health through a variety of mechanisms of action, mainly through antioxidant and anti-inflammatory action, activating GABA-mediated protective mechanisms, reducing pain, and promoting myelination through neurotrophic regulatory factors.

According to a preferred embodiment, the combination of the invention consists of PEA and *Equisetum* in a PEA/*Equisetum* weight ratio (at 10% in silica) ranging from 1/1 to 10/1, preferably from 2/1 to 4/1.

It is evident that when an *Equisetum* having a silica titer different than 10% is used in the combination of the invention, the ratio can be calculated from the above ratio according to the silica titer. The same calculation should be made when using an *Equisetum* in liquid form; in this case, an amount of *Equisetum,* such as at the rate of drops of mother tincture or macerate, should be calculated to provide an equal amount of silica.

The combination of the invention has been shown to be well tolerated, nontoxic, and therefore can be used in therapy.

It was also surprisingly found that the combination with *Equisetum* promotes PEA absorption, solving the problem of low bioavailability without at the same time altering intestinal permeability.

According to another of its aspects, it is an object of the invention a food, nutraceutical and/or pharmaceutical composition comprising the combination of PEA and *Equisetum,* as defined above, together with one or more physiologically acceptable excipients and/or vehicles.

According to another of its aspects, it is an object of the invention a food, nutraceutical and/or pharmaceutical composition comprising the combination of PEA and *Equisetum,* as defined above, together with one or more physiologically acceptable excipients and/or vehicles, for use in the protection and regeneration of the peripheral nervous system, in a mammal. Said mammal is preferably the human being.

According to a preferred embodiment, the composition for the use of the invention comprises from 100 mg to 1,500 mg, preferably from 300 mg to 1,200 mg of PEA and from 50 mg to 1,000 mg, preferably from 150 mg to 600 mg of *Equisetum* (e.g., at 10% in silica).

According to a preferred embodiment, the composition for use of the invention comprises 300 mg of PEA and 150 mg of *Equisetum* (e.g., at 10% in silica).

According to a preferred embodiment, the composition for use of the invention comprises 600 mg of PEA and 300 mg of *Equisetum* (e.g., at 10% in silica)

According to a preferred embodiment, the composition for use of the invention comprises 300 mg of PEA 80 mesh and 150 mg of *Equisetum* (e.g., at 10% in silica).

According to a preferred embodiment, the composition for use of the invention comprises 600 mg of PEA 80 mesh and 300 mg of *Equisetum* (e.g., at 10% in silica).

If desired or necessary, the composition of the invention may also contain additional active ingredients, preferably of natural origin, useful for the prevention and treatment of the nervous diseases described herein.

According to a preferred embodiment, the compositions of the invention are oral or buccal, solid, semisolid, gel, or liquid compositions and, as mentioned, contain physiologically acceptable excipients and/or conventional vehicles.

Compositions of the invention may be in the form of tablets, hard capsules, soft capsules, granules, fine granules, powders, tablets, syrups, emulsions, suspensions and solutions suitable for oral administration. Other suitable food, nutraceutical and/or pharmaceutical forms may also be used.

Compositions of the invention are oral compositions, preferably solid.

Such compositions can be taken alone or with water or, especially when in the form of powders or granules, can be mixed with other foods, such as with yoghurt, creams, gels, and the like.

Alternatively, the compositions of the invention can be in ready-to-use drinkable form, such as in the form of drinking sachets, of the "stick pack" type, in this case preferably in cream or gel form.

The types of food, nutraceutical and/or pharmaceutical additives used in preparing the compositions of the invention, the content ratios of the additives to the active ingredients, and the methods of preparing the food, nutraceutical and/or pharmaceutical composition may be appropriately selected by the person skilled in the art.

For conventional vehicles and excipients, organic or inorganic substances, or solid or liquid substances may be used as excipients and vehicles, provided they are edible, physiologically acceptable, and compatible with all other components of the composition.

As mentioned, the person skilled in the art is perfectly capable of selecting the most suitable vehicles and excipients for the preparation of the composition.

As examples, organic or inorganic substances, or solid or liquid substances may be used as excipients and vehicles, provided they are edible and pharmaceutically acceptable. Examples of excipients used in the preparation of solid food, nutraceutical, and/or pharmaceutical compositions include, for example, lactose, sucrose, starch, talc, cellulose, dextrins, kaolin, calcium carbonate, stearic acid or magnesium stearate, lactose, polyethylene glycol, mannitol, sorbitol, chelating agents, anti-caking agents, sweetening agents, preservative agents, and flavoring agents.

For the preparation of liquid compositions for oral administration, a conventional inert diluent such as water or an oil, such as a vegetable oil, may be used. The liquid composition may contain, in addition to the inert diluent, auxiliaries such as wetting agents, suspending agents, sweeteners, flavorings, colorants, and preservatives. The liquid composition may be included in capsules of an absorbable material, such as gelatin.

Sweeteners may be one or more natural sugars, optionally reduced, such as sucrose, dextrose, xylitol, mannitol, or sorbitol, or a synthetic product such as sodium saccharin, aspartame, acesulfame k, or sucralose. Acidifying agents may also be added.

Flavoring agents are food-acceptable, nutraceutical and/or pharmaceutical flavors and tastes of synthetic oils or natural oils, the latter extracted from plants, flowers, fruits and their combinations, such as cinnamon, mint, anise, and citrus leaves, bitter almonds, citrus fruits, especially orange and/or lemon oil, tilia, vanilla, chocolate and grapefruit. Chocolate, vanilla or eucalyptus flavorings and fruit essences, especially apple, pear, peach, strawberry, apricot, orange, lemon and grape, can also be used advantageously.

The combination of the invention, preferably in the form of a composition as defined above, may be administered once or several times a day, preferably once or twice a day. The dosage and daily dose may of course vary according to the age, sex and health status of the subject to be treated as well as the severity of the disease and the type of therapy, preventive or curative.

The combination and composition of the invention provide an effective natural therapeutic response to damage to the nervous system, particularly the peripheral nervous system, by providing protection and neuronal regeneration, and are a viable alternative to conventional drugs.

The combination of the invention has undergone numerous experimental tests to assay its safety and efficacy. These tests have shown that the combination is nontoxic and is well tolerated, even at high dosages. In addition, tests have shown that the combination has an important action in preventing damage related to peripheral neuronal degeneration, acting mainly through anti-inflammatory and antioxidant mechanisms, and is therefore useful in preventing and repairing nerve damage, especially peripheral nerve damage.

In fact, the combination combats the production of inflammatory markers that are the cause of PNS neuron degeneration and aging, is active on the balance of ROS ("reactive oxygen species") to prevent their accumulation and the consequent acceleration of nerve damage, improves neuronal viability and survival by inducing the production of neuroprotective factors and activating neurogenic mechanisms.

Details of the tests as well as the results obtained are given in the Experimental Section below.

According to another of its aspects, it is an object of the invention a kit that comprises

- at least one food, nutraceutical and/or pharmaceutical composition comprising PEA together with one or more physiologically acceptable excipients or vehicles; and
- at least one food, nutraceutical and/or pharmaceutical composition including *Equisetum,* as defined above, together with one or more physiologically acceptable excipients or vehicles.

The two compositions can be in any food, nutraceutical, and/or pharmaceutical form, independently of each other, and are compositions suitable for oral administration.

According to one embodiment, the kit of the invention includes from 5 to 60 compositions of PEA and from 5 to 60 compositions of *Equisetum,* e.g., from 10 to 30 compositions of each of the two components. The two formulations can be taken simultaneously or sequentially, advantageously they are administered simultaneously or at least within a short period of time so that they can exert a synergistic effect.

According to one embodiment, the kit of the invention also includes an instruction leaflet.

The preferred embodiments set forth above as well as the dosages are also applicable to the kit of the invention. According to another of its aspects, it is an object of the invention a method for neuronal protection and regeneration, preferably of the peripheral nervous system, which comprises administering an effective dose of the combination or, preferably, the composition of the invention as defined above to a subject in need thereof.

FRn embodiments of the present invention are given below.

FR1. A food, nutraceutical and/or pharmaceutical combination that comprises or alternatively consists of a palmitoylethanolamide (PEA) and an *Equisetum.*

FR2. The combination according to FR1, characterized by the fact that said *Equisetum* is *Equisetum Arvense.*

FR3. The combination according to FR1 or FR2, characterized by the fact that the weight ratio of palmitoylethanolamide/Equisetum is from 1/1 to 10/1, preferably from 2/1 to 4/1; said ratio being based by considering *Equisetum* in powder form titrated in silica in a percentage from 1% to 20%, preferably from 5% to 15%, even more preferably from 7% to 10% in silica.

FR4. The combination according to any one of FR1-3, wherein said palmitoylethanolamide is palmitoylethanolamide having preferably an average fineness or size or average particle size ranging from 20 mesh to 120 mesh, preferably from 40 mesh to 100 mesh, even more preferably from 60 mesh to 80 mesh.

FR5. The combination according to any one of FR1-4, wherein said combination comprises PEA from 100 mg to 1,500 mg, preferably from 300 mg to 1,200 mg, and *Equisetum* from 50 mg to 1,000 mg, preferably from 150 mg to 600 mg, said amount being based considering *Equisetum* in powder form preferably titrated at 10% in silica. FR6. The combination according to FR5, wherein said combination comprises 300 mg of PEA and 150 mg of *Equisetum,* preferably comprises 600 mg of PEA and 300 mg of *Equisetum.*

FR7. The combination according to any one of FR1-6, wherein said combination is (i) for use in a method for the neuronal protection and regeneration of the peripheral nervous system and/or (ii) for use in method for the prevention and repair of damage to the peripheral nervous system.

FR8. A food, nutraceutical, and/or pharmaceutical composition comprising the combination according to any one of FR1-7, preferably together with one or more food-grade or pharmaceutical grade excipients, and/or physiologically acceptable vehicles; preferably said composition is (i) for use in a method for the neuronal protection and regeneration of the peripheral nervous system and/or (ii) for use in method for the prevention and repair of damage to the peripheral nervous system.

FR9. The composition according to FR8, wherein said composition is suitable for oral administration.

FR10. A kit that comprises (a) at least one food, nutraceutical, and/or pharmaceutical composition comprising PEA according to any one of FR1-7, preferably together with one or more physiologically acceptable excipients or vehicles, and (b) at least one food, nutraceutical, and/or pharmaceutical composition comprising *Equisetum* according to any one of FR1-7, preferably together with one or more physiologically acceptable excipients or vehicles.

**Experimental Section**

Examples of compositions

Example 1

A composition is prepared in capsule form comprising:

| | |
|---|---|
| - PEA | 300mg |
| - *Equisetum arvense* powder with titer at 10% in silica | 150mg |

together with excipients and/or conventional vehicles.

Example 2

A composition is prepared in tablet form comprising:

| | |
|---|---|
| - PEA | 600mg |
| - *Equisetum arvense* powder with titer at 10% in silica | 300mg |

together with excipients and/or conventional vehicles.

EXPERIMENTAL TESTS

In the following tests and Figures:

- the combination of the invention is called "EquiPEA™";
- the comparative compound is Normast® containing 600 mg of ultramicronized palmitoylethanolamide (PEA);
- PEAm indicates micronized PEA;
- PEAum indicates ultramicronized PEA;
- min indicates minute/minutes;
- h indicates hour(s).

Data reported in the tests are expressed as mean+/- SD of 5 independent experiments conducted in triplicate. For all tested forms, the results obtained between 1 hour and 5 hours were statistically significant over control (p<0.05).

ABSORPTION AND BIOAVAILABILITY

Transwell permeable supports were used, which are convenient and easy-to-use devices for permeability studies. Cells are cultured for 21 days, time required to polarize and differentiate by forming a continuous epithelium (monolayer) with functional tight junctions and microvilli at the apical level (brush rim). The cell monolayer provides a physical and biochemical barrier to the passage of ions and small molecules through two compartments: one apical, (human intestinal lumen), and one basolateral (blood circulation at pH7.4). These mechanisms and correlations of drug interaction, cell differentiation, and cell signaling in these 3-D environments mimic the biochemical characterization of intestinal tissue, reducing here the adoption of other methods to assess absorption of active ingredients for oral intake. Drug safety and efficacy studies are efficient and relatively easier to perform in 3D cultures, reducing the time required. The convenience of handling cells in vitro while obtaining results that reflect in vivo conditions, avoiding ethical concerns about animal use, is making CaCo2/HT29 co-culture techniques suitable for assessing the degree of permeability while excluding irritability or damage to the intestinal epithelium. This model was performed by the Transwell technique using selected intestinal cells (at mature stage and after microvilli formation) seeded on the apical side of the insert in defined 70/30 proportions cultured for 28 days (Hoffmann P et al., 2021.https://doi.org/10.1371/journal.pone.0257824).

This method takes advantage of a co-culture model of enterocytes (Caco-2) and mucosecreting cells (HT29-MTX) by recreating the two major pathways of human intestinal uptake: passive diffusion, through the paracellular/-transcellular pathway, and active transport mediated by various transporters, depending on the molecule of interest, into the bloodstream and then directly to the target. The cell uptake assay on Transwell provides an in-depth analysis of the ability of cells to sense a particular chemo-attractant and migrate through a physical barrier toward it. The use of a co-culture is important to best mimic the human condition by creating a seemingly perfect environment. This model makes it possible to recreate the intestinal epithelium in vitro in the most physiological way possible (Reale O. et al., 2020. doi.org/10.1016/j.chemosphere.2020.128497)

PHASE 1

Tests performed

- Treatment for 30 min-6h for absorption at small intestine level
- Toxicity analysis (product safety)
- Analysis of ROS production
- Absorption and bioavailability (TEER and Papp analysis; Morsanuto V, et al. 2020.doi: 10.3390/brainsci10070457)

- Tight junction (TJ) analysis by kit
- Statistical analysis of data (5 independent experiments conducted in triplicate)

TESTED SUBSTANCES AND DOSAGES

Table 1

| PEA um | 200mg/300mg/600mg/1200mg/1800mg |
|---|---|
| PEA m | 200mg/300mg/600mg/1200mg/1800mg |
| PEA 80mesh | 200mg/300mg/600mg/1200mg/1800mg |
| PEA 20/40mesh | 200mg/300mg/600mg/1200mg/1800mg |
| PEA crystals | 200mg/300mg/600mg/1200mg/1800mg |
| Normast® | 200mg/300mg/600mg/1200mg/1800mg |
| EquiPEA™ | PEA+50% (W/W) *Equisetum* at 10% in silica dosage |
| EquiPEA™ | PEA+1/4 (W/W) *Equisetum* at 10% in silica dosage |
| *Equisetum* | at 10% in silica at 150mg/300mg/600mg |

ABSORPTION

Evaluation of passage through the intestinal barrier with fluorescent tracer

Figure 1

An analysis of different forms of PEA over time (from 30 min to 6h) was conducted to determine differences in absorption necessary for the choice of PEA to be used in EquiPEA™, comparing the data with a commercial product (Normast®) at the same dosage (200mg). Among the forms of PEA it can be seen that PEA crystals results to have low absorption compared to all forms tested ($p<0.05$) and also compared to Normast® ($p<0.05$). Between the micronized and ultra-micronized forms, no substantial differences can be observed even compared to Normast® ($p>0.05$).
Between 80 and 40 mesh, 80 mesh seems to have a profile more similar to Normast®. All forms show a similar trend over time with peak absorption around 3 hours. This trend appears to be in line with the trend of the commercially available product.

Figure 2

An analysis of different forms of PEA over time (from 30 min to 6h) was conducted to determine differences in absorption necessary for the choice of PEA to be used in EquiPEA™, comparing the data with a commercial product (Normast®) at the same dosage (300mg). Among the forms of PEA it can be seen that PEA crystals results to have low absorption compared to all forms tested ($p<0.05$) and also compared to Normast® ($p<0.05$). Between micronized and ultra-micronized forms, no substantial differences can be observed but the peak at 3h is lower than Normast® for both forms. Between 80 and 40 mesh, 80 mesh seems to have a profile more similar to Normast®, with a greater peak at 3h ($p<0.05$). All forms show a similar trend over time with absorption peak around 3h. This trend appears to be in line with the trend of the commercially available product.

Figure 3

An analysis of different forms of PEA over time (from 30 min to 6h) was conducted to determine differences in absorption necessary for the choice of PEA to be used in EquiPEA™, comparing the data with a commercial product (Normast®) at the same dosage (600mg). Among the forms of PEA it can be seen that PEA crystals results to have low absorption compared to all forms tested ($p<0.05$) and also compared to Normast® ($p<0.05$). Between the micronized and ultra-micronized forms, the ultra form appears to be more similar with Normast® with a peak at 3h slightly lower than Normast®. Between 80 and 40 mesh, 80 mesh appears to have a profile more similar to Normast®, with a peak at 3h slightly higher ($p<0.05$). Analysis of different forms of PEA over time (from 30 min to 6h) to determine differences in absorption necessary for the choice of PEA to be used in EquiPEA™ comparing the data with a commercial product (Normast®) at the same dosage (300mg). Among the forms of PEA it can be seen that PEA crystals results to have low absorption compared to all forms tested ($p<0.05$) and also compared to Normast® ($p<0.05$). Between micronized and ultra-micronized forms, no substantial differences can be observed but the peak at 3h is lower than Normast® for both forms. Between 80 and 40 mesh, 80 mesh seems to have a profile more similar to Normast®, with a greater peak at 3h ($p < 0.05$). All forms show a similar trend over time with absorption peak around 3h. This trend appears to be in line with the trend of the commercially available product.

Figure 4

An analysis of different forms of PEA over time (from 30 min to 6h) was conducted to determine differences in absorption necessary for the choice of PEA to be used in EquiPEA™, comparing the data with a commercial product (Normast®) at the same dosage (1200mg). Among the forms of PEA, it can be seen that PEA crystals always results to have low absorption compared to all forms tested ($p < 0.05$) and also compared to Normast® ($p < 0.05$). Between the micronized and ultra-micronized forms, the ultra form appears to be more similar to Normast® with a peak at 3h overlapping with Normast®. Between 80 and 40 mesh, 80 mesh shows a profile more similar to Normast®, with an almost overlapping 3h peak. All forms show a similar trend maintaining the absorption peak around 3h. This trend appears to be in line with the trend of the commercially available product.

Figure 5

An analysis of different forms of PEA over time (from 30 min to 6h) was conducted to determine differences in absorption necessary for the choice of PEA to be used in EquiPEA™, comparing the data with a commercial product (Normast®) at the same dosage (1800mg). Among the forms of PEA, it can be seen that PEA crystals always results to have low absorption compared to all forms tested ($p < 0.05$) and also compared to Normast® ($p < 0.05$). Between the micronized and ultra-micronized forms, the ultra form appears to overlap with Normast® with a peak at 3h. Between 80 and 40 mesh, 80 mesh shows a profile more similar to Normast®, with a slightly lower peak at 3h. All forms show a similar trend maintaining the absorption peak around 3h. This trend appears to be in line with the trend of the commercially available product.

ABSORPTION/BIOAVAILABILITY

Analysis of permeability and plasma bioavailability are shown in Table 2 and 3.

|  | 200mg | 300mg | 600mg | 1200mg | 1800mg |
|---|---|---|---|---|---|
| PEA Crystals | 25% | 28% | 33% | 32% | 29% |
| PEA M | 53% | 59% | 65% | 64% | 62% |
| PEA UM | 53% | 59% | 65% | 64% | 62% |
| PEA 40 Mesh | 50% | 56% | 62% | 61% | 58% |
| PEA 80 Mesh | 54% | 60% | 66% | 65% | 63% |
| Normast | 52% | 58% | 64% | 63% | 60% |

Table 2.

PERMEABILITY:

Values are derived from the application of the formula for calculating permeability

$$Jmax\ [C]/(Kt + [C])$$

C: initial probe concentration
Jmax: fluorescence transition.
Kt: Michaelis-Menten constant
Morsanuto V, et al. 2020. doi:10.3390/brainsci10070457

|  | 200mg | 300mg | 600mg | 1200mg | 1800mg |
|---|---|---|---|---|---|
| PEA Crystals | $1.24 \times 10^{-6}$ | $1.29 \times 10^{-6}$ | $1.34 \times 10^{-6}$ | $1.33 \times 10^{-6}$ | $1.30 \times 10^{-6}$ |
| PEAm | $1.50 \times 10^{-6}$ | $1.56 \times 10^{-6}$ | $1.64 \times 10^{-6}$ | $1.63 \times 10^{-6}$ | $1.60 \times 10^{-6}$ |
| PEAum | $1.50 \times 10^{-6}$ | $1.56 \times 10^{-6}$ | $1.64 \times 10^{-6}$ | $1.63 \times 10^{-6}$ | $1.60 \times 10^{-6}$ |
| PEA 40 Mesh | $1.47 \times 10^{-6}$ | $1.58 \times 10^{-6}$ | $1.66 \times 10^{-6}$ | $1.64 \times 10^{-6}$ | $1.61 \times 10^{-6}$ |
| PEA 80 Mesh | $1.52 \times 10^{-6}$ | $1.62 \times 10^{-6}$ | $1.73 \times 10^{-6}$ | $1.68 \times 10^{-6}$ | $1.65 \times 10^{-6}$ |
| Normast | $1.49 \times 10^{-6}$ | $1.55 \times 10^{-6}$ | $1.63 \times 10^{-6}$ | $1.60 \times 10^{-6}$ | $1.58 \times 10^{-6}$ |

Table 3.

PLASMA BIOAVAILABILITY:

These values are derived from application of the formula for calculating Papp (Biganzoli E, 1999. doi:10.1016/s0014-827x(99)00069-5).

Papp values $<0.2 \times 10^6$ cm/s indicate very poor absorption with <1% bioavailability. Data between $0.2 \times 10^6$ and $2 \times 10^6$ cm/s indicate a bioavailability between 1 and 90%.

Conclusions:

- PEA concentration remains relevant for absorption up to 1200 mg
- the form of PEA is important for its absorption.

Among the forms of PEA, at all concentrations, PEA 80mesh appears best, maintaining levels similar to Normast® with peak at 3h.

The effect of PEA 80mesh from 300mg to 1200 mg is comparable to the absorption and effects of Normast , but at concentrations from 300 mg to 1200 mg.

PEA 80mesh vs Normast® from 300 to 1200 was further tested.

Unless a plasma peak of at least 20 pmol/mL is reached, PEA is unable to reach inflamed tissues in useful concentrations and is therefore inactive.

The plasma concentration achieved was more than 20 pmol/ml with PEA 80 mesh, Normast®, PEAm, PEAum. The effective dose is about 600 mg/ml.

TOXICITY

The cell viability of intestinal epithelium over time was assessed to exclude toxicity.

Tests conducted:

- Analysis of PEA 80mesh at different concentrations (300-600-1200 mg) compared with Normast® at the same dosage (300-600-1200 mg) measured at 3h (absorption peak) and at 6h (end of absorption stimulation in the small intestine)
- Assessment of the viability of increasing concentrations of *Equisetum* (150-300-600 mg) titrated at 10% in silica
- Assessment of the viability of increasing concentrations of PEA 80 mesh (300-600-1200 mg) combined with *Equisetum* (150-300-600 mg) titrated at 10% in silica

PEA 80 mesh and Normast®, at all concentrations tested, are found to increase cell viability while allowing toxicity to be excluded in their use on intestinal epithelium. Viability follows the trend observed for absorption, supporting its permeability physiology. *Equisetum* shows a safety profile at all concentrations tested. The combination of EquiPEA™ with different concentrations of PEA and *Equisetum* shows that the safest are 300mg+150mg/600mg+300mg/1200+300 or 600mg *(PEA/Equisetum)* (Tables 4 to 7).

| Normast | | |
|---|---|---|
| | 300mg | 600mg | 1200mg |
| 3h | 80 ± 1.71 | 90 ± 1.21 | 72 ± 1.46 |
| 6h | 76 ± 1.48 | 87 ± 1.67 | 70 ± 1.11 |

Table 4.

| PEA 80mesh | | |
|---|---|---|
| | 300mg | 600mg | 1200mg |
| 3h | 85 ± 0.83 | 92 ± 1.23 | 70 ± 1.48 |
| 6h | 78 ± 1.74 | 90 ± 1.26 | 68 ± 1.27 |

Table 5.

| Equisetum 10% | | |
|---|---|---|
| | 150mg | 300mg | 600mg |
| 3h | 45 ± 0.85 | 49 ± 1.48 | 46 ± 1.57 |
| 6h | 30 ± 1.27 | 44 ± 1.22 | 40 ± 1.57 |

Table 6.

| 3h | | PEA 80mesh | | |
|---|---|---|---|---|
| | | 300mg | 600mg | 1200mg |
| EQUISETUM 10% | 150mg | 87 ± 1.33 | 74 ± 1.26 | 68 ± 1.13 |
| | 300mg | 72 ± 1.25 | 94 ± 0.86 | 74 ± 1.01 |
| | 600mg | 56 ± 1.07 | 67 ± 1.28 | 75 ± 1.36 |

Table 7.

EVALUATION OF ROS PRODUCTION IN THE INTESTINAL EPITHELIUM OVER TIME TO EXCLUDE IRRITABILITY.

Tests conducted:

- Analysis of PEA 80mesh at different concentrations (300-600-1200 mg) compared with Normast® at the same dosage (300-600-1200 mg) measured at 3h (absorption peak) and at 6h (end of absorption stimulation in the small intestine)
- ROS evaluation of increasing concentrations of *Equisetum* (150-300-600 mg) titrated at 10% in silica
- ROS evaluation of increasing concentrations of PEA 80mesh (300-600-1200 mg) combined with *Equisetum* (150-300-600 mg) titrated at 10% in silica

PEA 80 mesh and Normast® at all concentrations tested show no significant increase in ROS production (p>0.05) compared with control, supporting their safety of use. *Equisetum* shows antioxidant property (p<0.05) compared to control at all concentrations. The EquiPEA™ combination with different concentrations of PEA and *Equisetum* confirms what was observed with cell viability 300mg+150mg/600mg+300mg/1200+300 or 600mg *(PEA/Equisetum)* (Tables 8 to 11).

| Normast | 300mg | 600mg | 1200mg |
|---|---|---|---|
| 3h | 4 ± 1.14 | 5 ± 1.67 | 7 ± 1.48 |
| 6h | 8 ± 1.15 | 7 ± 1.56 | 10 ± 1.22 |

Table 8.

| PEA 80mesh | 300mg | 600mg | 1200mg |
|---|---|---|---|
| 3h | 3 ± 1.78 | 2 ± 1.74 | 1 ± 1.19 |
| 6h | 7 ± 1.41 | 6 ± 1.38 | 5 ± 1.47 |

Table 9

| Equisetum 10% | 150mg | 300mg | 600mg |
|---|---|---|---|
| 3h | -8 ± 1.74 | -9 ± 1.46 | -9 ± 1.71 |
| 6h | -9 ± 1.38 | -9 ± 1.17 | -9 ± 1.48 |

Table 10.

| 3h | | PEA 80mesh | | |
|---|---|---|---|---|
| | | 300mg | 600mg | 1200mg |
| EQUISETUM 10% | 150mg | -14 ± 1.13 | -13 ± 0.86 | -11 ± 1.77 |
| | 300mg | -14 ± 1.01 | -15 ± 0.89 | -14 ± 1.15 |
| | 600mg | -10 1.36 | -11 ± 1.23 | -13 ± 1.48 |

Table 11.

Conclusions:

- PEA 80mesh and *Equisetum* are safe for intestinal epithelium
- PEA 80mesh combined with 10% *Equisetum* is able to acquire antioxidant properties while maintaining positive cellular vitality
- oxidative stress can be excluded from their use

It seems evident that the effect of PEA 80mesh 300mg to 1200mg combined with *Equisetum* 150mg to 300mg is comparable or even superior to the effects of Normast®.

The combination of PEA 80mesh 300 to 1200mg combined with *Equisetum* 150 mg to 600 mg was further tested vs Normast® .

ABSORPTION

Tests conducted:

- Evaluation of passage across the intestinal barrier with fluorescent tracer
- Analysis of PEA 80mesh over time (from 30 min to 6h) combined with *Equisetum* comparing data with a commercial product (Normast®) at the same dosage (300mg to 1200mg)

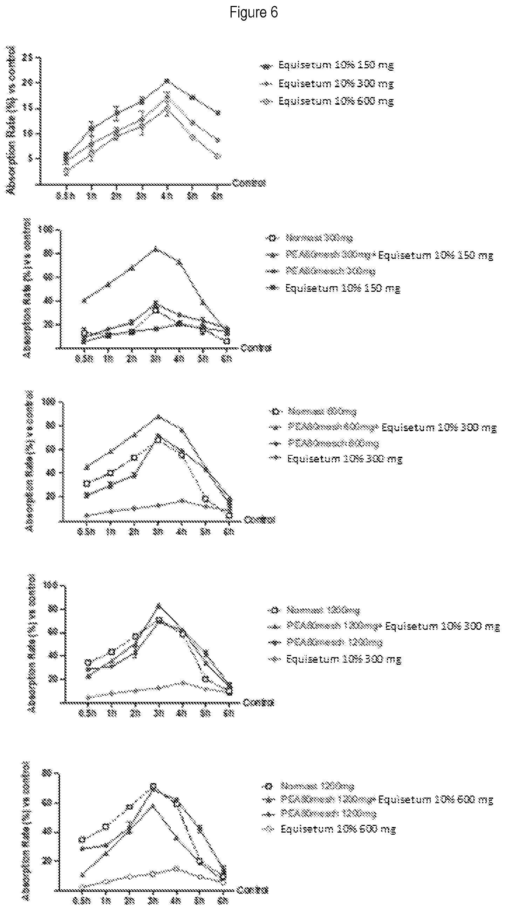

From Figure 6 it can be seen that PEA 80mesh combined with *Equisetum* is able to enhance the absorption of PEA 80mesh leading the values to be in some cases even greater than the commercial product (Normast®). EquiPEA™ also increases the timing of absorption: the peak at 3h is found to be maintained very high even at 4h, and the increase begins to be observed even at 2h. The combination of *Equisetum* 600mg and PEA 80mesh 1200mg seems to be excessive such that the absorbed portion no longer increases.

TEER

Evaluation of transepithelial resistance to determine that intestinal tissue integrity is preserved.

Test performed:

- Analysis of PEA 80mesh over time (from 30 min to 6h) combined with *Equisetum* comparing data with a commercial product (Normast®) at the same dosage (300mg to 1200mg)

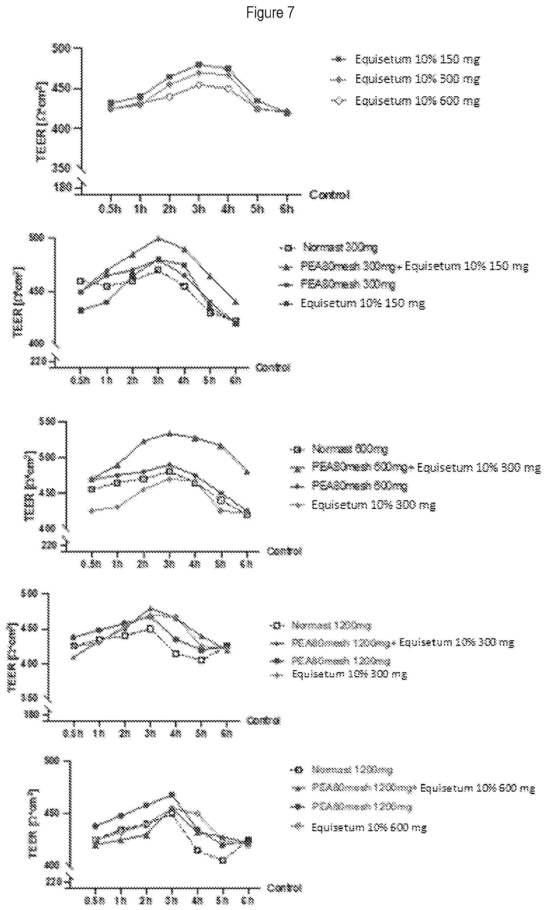

The analysis supports the observed absorption, also in terms of intestinal wall permeation kinetics. From Figure 7 it can be observed that PEA 80mesh combined with *Equisetum* is able to enhance the absorption of PEA 80mesh leading the values to be in some cases even higher than the commercial product (Normast®). The peak at 3h is found to be maintained very high even at 4h, and the increase begins to be observed even at 2h. The combination with *Equisetum* 600mg and PEA 80mesh 1200mg seems to be excessive such that TJS does not increase more work. No permeability alteration is detected (basal value is preserved).

PERMEABILITY ANALYSIS (Table 12)

Values are derived from application of the formula for calculating permeability (Morsanuto V, et al, 2020 doi:10.3390/brainsci10070457)

| Subtances | Absorption % |
|---|---|
| Normast 300mg | 58% |
| PEA 80 Mesh 300mg + Equisetum 10% 150 mg | 89% |
| PEA 80mesh 300mg | 60% |
| Equisetum 10% 150 mg | 14% |
| Normast 600mg | 64% |
| PEA 80 Mesh 600mg + Equisetum 10% 300 mg | 93% |
| PEA 80mesh 600mg | 66% |
| Equisetum 10% silica 300 mg | 27% |
| Normast 1200mg | 63% |
| PEA 80 Mesh 1200mg + Equisetum 10% 300 mg | 83% |
| PEA 80 Mesh 1200mg + Equisetum 10% 600 mg | 67% |
| PEA 80mesh 1200mg | 65% |
| Equisetum 10% silica 600 mg | 37% |

Table 12

PLASMA BIOAVAILABILITY (Table 13)

Values are derived from the application of the formula for calculating Papp

- Papp values <$0.2\times10^6$ cm/s indicate very poor absorption with <1% bioavailability,
- the data between $0.2\times10^{-6}$ and $2\times10^{-6}$ cm/s a indicate bioavailability between 1 and 90 percent, (Biganzoli E, 1999. doi:10.1016/s0014-827x(99)00069-5)

Conclusions:

- PEA 80mesh and *Equisetum* are safe for intestinal epithelium
- PEA 80mesh combined with *Equisetum* is able to increase transepithelial resistances suggesting that passage is mediated (paracellular)
- EquiPEA™ is able to increase the absorption of PEA

It is evident that the effect of PEA 80mesh from 300mg to 1200mg combined with *Equisetum* 150mg to 300mg is comparable or superior to the effects of Normast®.

PEA 80mesh from 300 to 1200mg combined with *Equisetum* from 150mg to 600mg were further tested vs Normast® .

EP 4 565 075 B1

| Subtances | Permeability values |
|---|---|
| Normast 300mg | $1.55 \times 10^{-6}$ |
| PEA 80 Mesh 300mg + Equisetum 10% 150 mg | $1.98 \times 10^{-6}$ |
| PEA 80mesh 300mg | $1.62 \times 10^{-6}$ |
| Equisetum 10% 150 mg | $0.28 \times 10^{-6}$ |
| Normast 600mg | $1.63 \times 10^{-6}$ |
| PEA 80 Mesh 600mg + Equisetum 10% 300 mg | $2.13 \times 10^{-6}$ |
| PEA 80mesh 600mg | $1.73 \times 10^{-6}$ |
| Equisetum 10% silica 300 mg | $0.54 \times 10^{-6}$ |
| Normast 1200mg | $1.60 \times 10^{-6}$ |
| PEA 80 Mesh 1200mg + Equisetum 10% 300 mg | $1.83 \times 10^{-6}$ |
| PEA 80 Mesh 1200mg + Equisetum 10% 600 mg | $1.62 \times 10^{-6}$ |
| PEA 80mesh 1200mg | $1.68 \times 10^{-6}$ |
| Equisetum 10% silica 600 mg | $0.75 \times 10^{-6}$ |

Table 13.

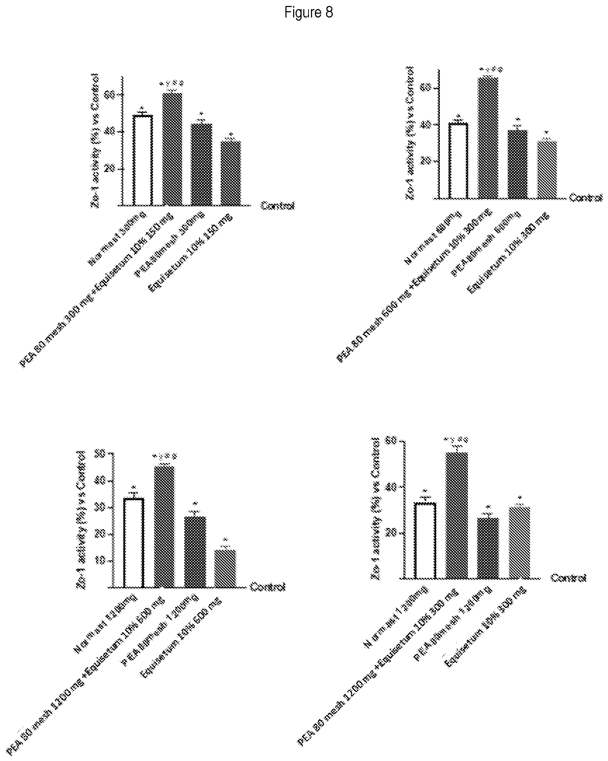

TJs (Figure 8)

Analysis of Zo-1 activity (mediates adhesion): Claudin (maintains structure) and Occludin (contributes to stabilization; Galla R, et al. 2021. doi:10.3390/biomedicines9111543)
The test is intended to determine barrier integrity and its functionality (avoid irritability or conditions that could lead to percolating bowel syndrome). Zo-1 analysis confirms the active role of TJs in the absorption of compounds. The analysis was conducted at 6h after the end of stimulation to verify that there were no alterations in intestinal structures The results show that the combination with *Equisetum* has a greater influence on zo-1, supporting its absorption.

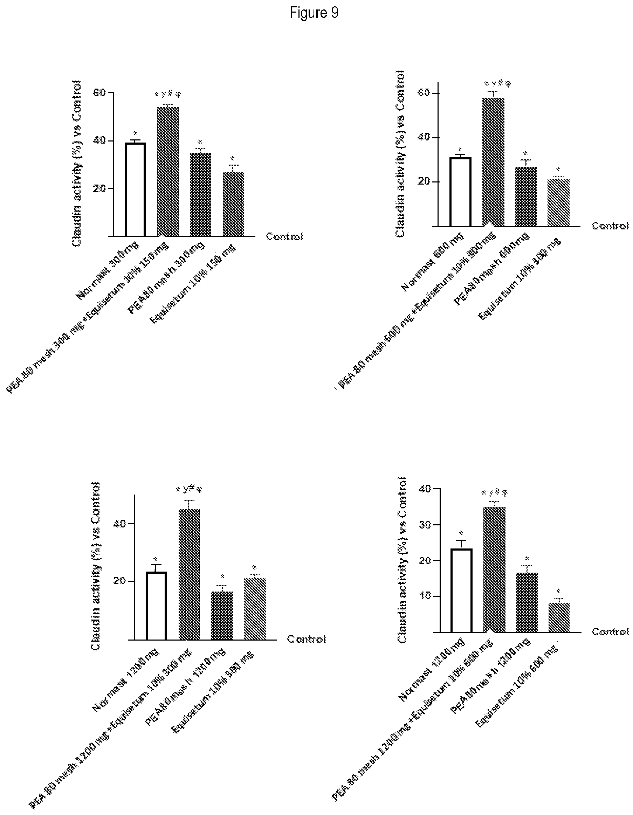

TJs (Figure 9)

Analysis of Zo-1 activity (mediates adhesion): Claudin (maintains structure) and Occludin (contributes to stabilization; Galla R, et al. 2021. doi:10.3390/biomedicines9111543)
The test is intended to determine barrier integrity and its functionality (avoid irritability or conditions that could lead to percolating bowel syndrome). Claudin analysis confirms the active role of TJs in the absorption of compounds. The analysis was conducted at 6h after the end of stimulation to verify that there were no alterations in intestinal structures. The results show that the combination with *Equisetum* has a greater influence on claudin, supporting its absorption.

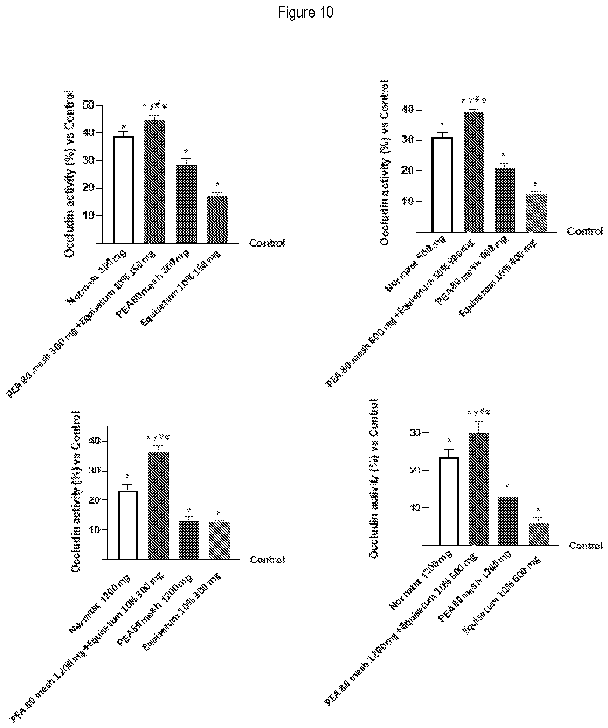

TJs (Figure 10)

Analysis of Zo-1 activity (mediates adhesion): Claudin (maintains structure) and Occludin (contributes to stabilization; Galla R, et al. 2021 doi:10.3390/biomedicines9111543)
The test is intended to determine barrier integrity and its functionality (avoid irritability or conditions that could lead to percolating bowel syndrome). Occludin analysis confirms the active role of TJs in the absorption of compounds. The analysis was conducted at 6h after the end of stimulation to verify that there were no alterations in intestinal structures. The results show that the combination with *Equisetum* has a greater influence on occludin, supporting its absorption.

Conclusions:

- PEA 80mesh and *Equisetum* are safe for intestinal epithelium

- They do not alter the permeability associated with opening/closing of tight junctions
- EquiPEA™ is able to increase PEA absorption through TJs also

It is evident that the effect of PEA 80mesh from 300mg to 1200mg combined with *Equisetum* from 150mg to 300mg is comparable or superior to the effects of Normast® .

PEA 80mesh from 300 to 1200mg associated with *Equisetum* from 150mg to 300mg were further tested in PHASE 2 vs Normast® .

PHASE 2

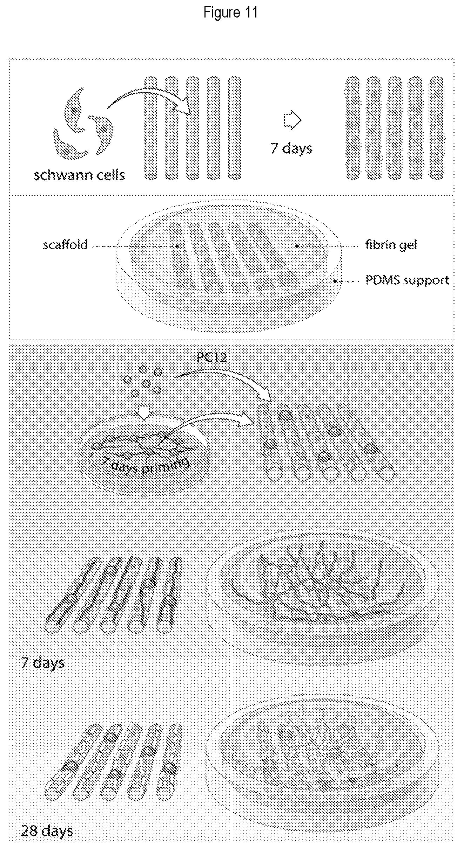

PNS *in vitro* model (Figure 11)

A motor neuron/Schwann cell (MN-SC) model was used to evaluate efficacy on the end target, excluding toxicity or irritability and promoting survival. It is a 3D model of a peripheral nerve containing aligned Schwann cells (RSC96) (green) encapsulated in a matrix that allows motor neurons (NSC34) to anchor on the surface (red) (Rayner et al. 2018. doi.org/10.1002/ar.23918). Such a model allows bridging between results obtained in *in vitro* models and more successful human studies, such that preclinical studies with 3D models are the basis for determining efficacy and safety as required by regulatory agencies, including, for example, the Medicines and Healthcare Products Regulatory Agency (MHRA) or the U.S. Food and Drug Administration (FDA).

The test examines any direct effects following intestinal passage on the peripheral nerve. Treating the MN-SC co-culture model with ascorbic acid induces the myelination process in the PNS, with increased expression of myelin markers. Treating with glial growth factor (neuregulin-1 isoform) the formation of myelin sheath can be blocked and demyelination induced (200 ng/ml of GGF from 14gg). It allows active control of myelinating processes and offers new opportunities to study pathophysiological processes (Park, et al. 2021. doi.org/10.1038/s41427-020-00273-w).

PHASE 2: Tests conducted

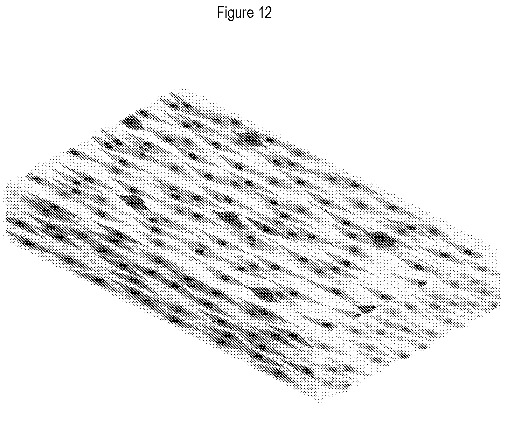

Motor neurons + Schwann cells (Figure 12, Schwann cells in green and motor neurons in red

Rayner, et al. 2007. doi.org/10.1002/ar.23918)

- Treatment for 24h to study the effect on PNS
- Toxicity analysis (product safety)
- Analysis of ROS production
- TNFa and IL2 inflammatory picture analysis
- Analysis of key markers involved in myelin sheath protection processes and neurite formation (NRG1, MPZ, p75 and ERB3, GABA A/B, endocannabinoid receptors
- NGF Assay

Statistical analysis of data (5 independent experiments conducted in triplicate)

TESTED SUBSTANCES AND DOSAGES:

Table 14

| | |
|---|---|
| Normast® | 300mg/600mg/1200mg |
| PEA 80mesh | 300mg/600mg/1200mg |
| *Equisetum* | 150/300mg/600mg 10% silica |
| EquiPEA ™ | PEA 80mesh 300mg + *Equisetum* (at 10% in silica) 150mg |
| EquiPEA ™ | PEA 80mesh 600mg + (at 10% in silica) 300mg |
| EquiPEA ™ | PEA 80mesh 1200mg + *Equisetum* (at 10% in silica) 300mg |

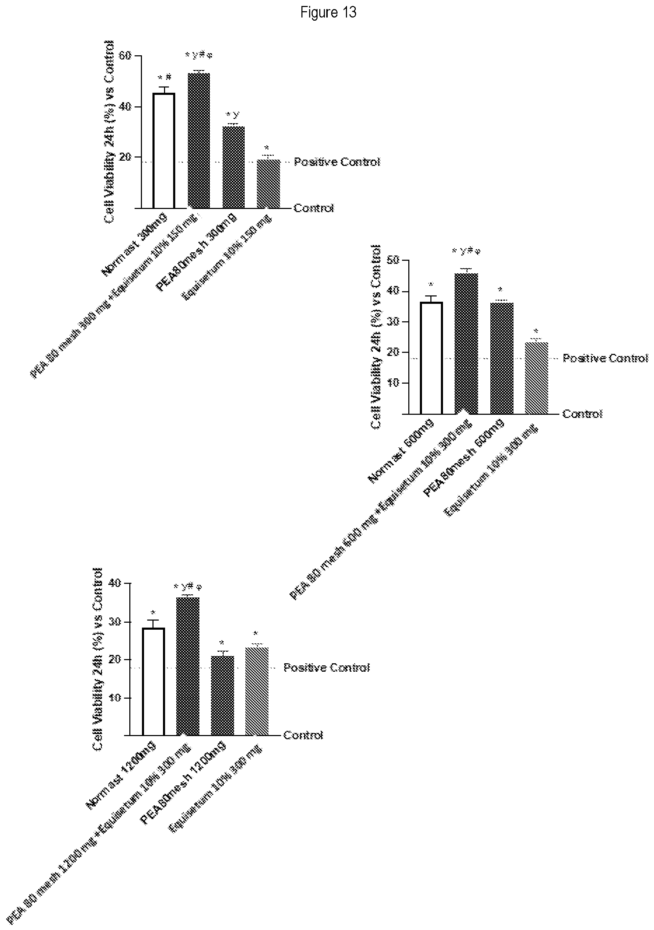

TOXICITY (Figure 13)

Evaluation of cell viability on the 3D PNS model and excluding toxicity

Tests conducted:

- Viability analysis of PEA 80mesh at different concentrations (300-600-1200 mg) compared with Normast® at the same dosage (300-600-1200 mg) after 24h of treatment
- Evaluation of the viability of increasing concentrations of *Equisetum* (150-300mg) titrated at 10% in silica
- Evaluation of the viability of increasing concentrations of PEA 80mesh (300-600-1200 mg) combined with *Equisetum* (150-300mg) titrated at 10% in silica

PEA 80 mesh and Normast®, at all concentrations tested, are found to increase cell viability allowing toxicity to be excluded in their use also on PNS after intestinal metabolism.

*Equisetum* shows a safety profile at all concentrations also tested on PNS after intestinal metabolism.

The combination of EquiPEA™ with different concentrations of PEA and *Equisetum,* confirms that the safest are 300mg+150mg/600mg+300mg/1200+300 or 600mg *(PEA/Equisetum)* also on PNS. The best performers seem to be 300+150mg and 600+300mg, also compared with Normast® ($p<0.05$).

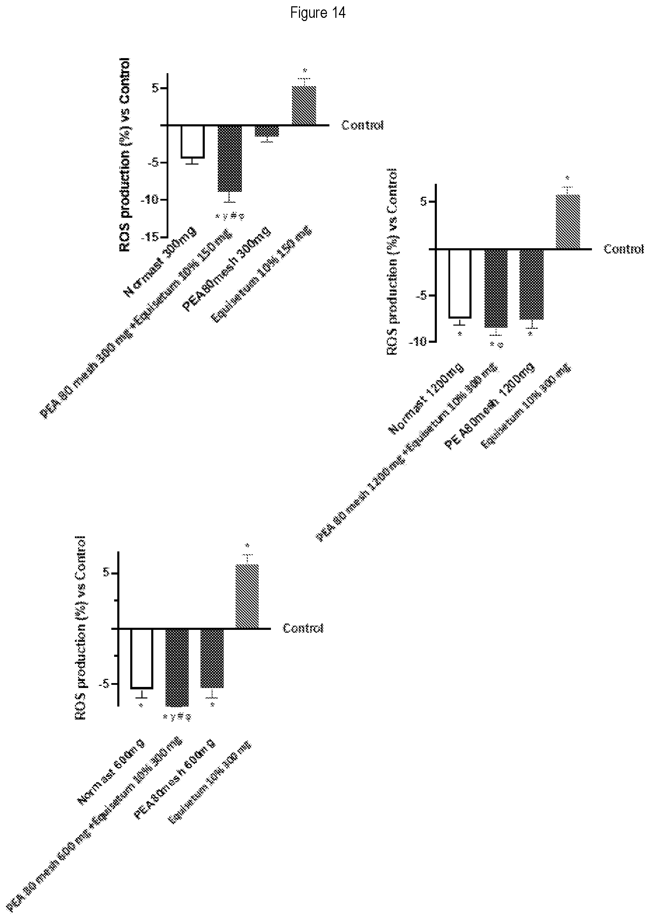

ROS PRODUCTION (Figure 14)

Evaluation of ROS production in terms of inhibition for reduction of oxidative stress related to nerve damage

Tests conducted:

- Analysis of PEA 80mesh-mediated ROS production at different concentrations (300-600-1200 mg) compared with Normast® at the same dosage (300-600-1200 mg) after 24h of treatment
- Evaluation of ROS at increasing concentrations of *Equisetum* (150-300 mg) titrated at 10% in silica
- Evaluation of ROS at increasing concentrations of PEA 80 mesh (300-600-1200 mg) combined with *Equisetum* (150-300 mg) titrated at 10% in silica

PEA 80 mesh and Normast® at all concentrations tested are found to reduce oxidative damage associated with demyelination. *Equisetum* fails to totally inhibit oxidation while having good antioxidant activity. The combination of EquiPEA™ with different concentrations of PEA and *Equisetum,* confirms that the most effective are 300mg+150mg/600mg+300mg/1200+300 or 600mg *(PEA/Equisetum)* under conditions of neuronal damage suggesting the importance of the association of the antioxidant and anti-inflammatory role.

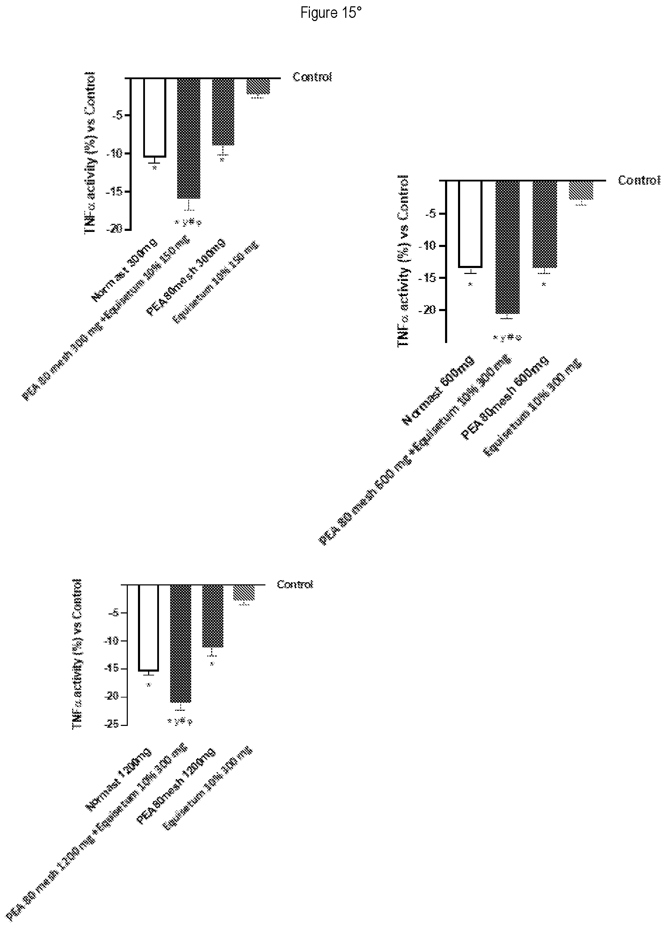

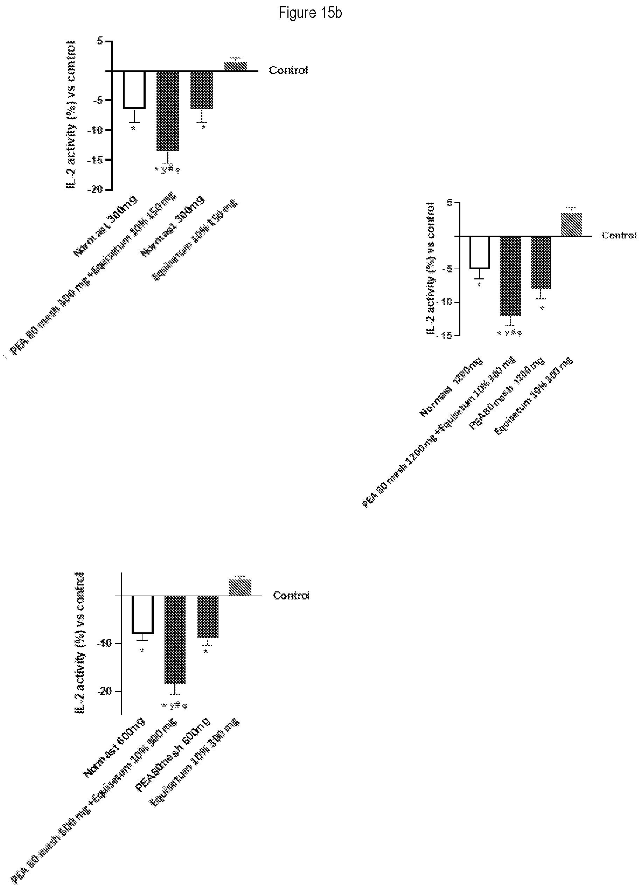

INFLAMMATION (Figure 15a and 15b)

Assessment of TNFa and IL-2 production to determine anti-inflammatory capacity

Tests conducted:

- Analysis of TNFa and IL-2 mediated by PEA 80mesh at different concentrations (300-600-1200 mg) compared with Normast® (300-600-1200 mg) after 24h of treatment
- Evaluation of TNFa and IL-2 at increasing concentrations of *Equisetum* (150-300mg) titrated at 10% in silica
- Evaluation of TNFa and IL-2 at increasing concentrations of PEA 80mesh (300-600-1200 mg) combined with *Equisetum* (150-300mg) titrated at 10% in silica

PEA 80 mesh and Normast® at all concentrations tested are found to reduce ($p<0.05$ vs control) inflammatory markers. *Equisetum* has less effect on markers, confirming its antioxidant role. The combination of EquiPEA™ with different concentrations of PEA and *Equisetum,* confirms that the most effective are 300mg+150mg/600mg+300mg/1200+300 or 600mg *(PEA/Equisetum)* under conditions of neuronal damage suggesting the importance of the anti-inflammatory role.

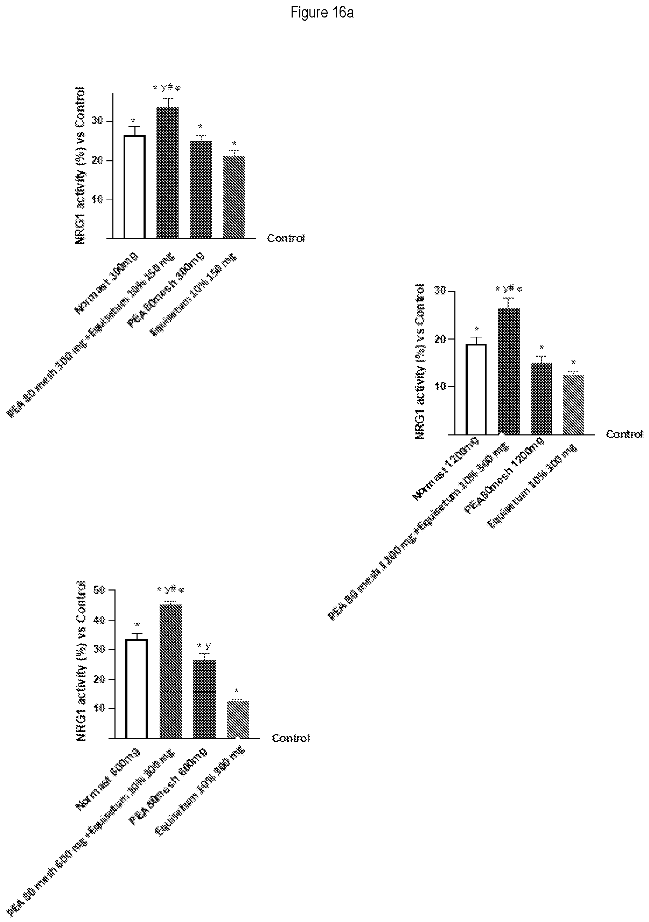

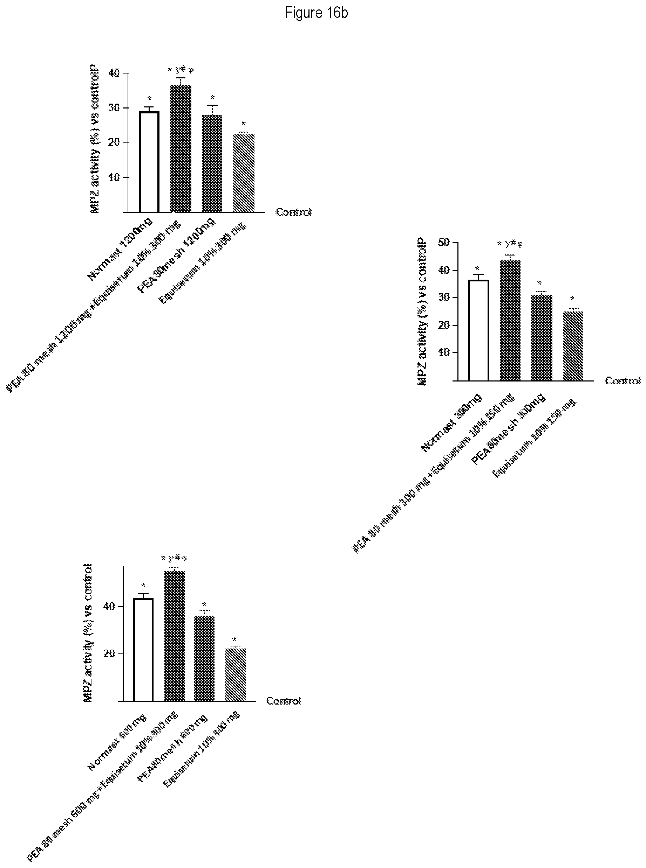

MARKERS (Figure 16a and 16b)

Evaluation of NGR1 and MPZ activity to determine myelination and compact myelin formation (Fricker et al. 2011, doi:10.2217/fnl.11.45; Wolbert J, et al. 2020. doi:10.1073/pnas.1912139117)

Tests conducted:

- Analysis of NRG1 and MPZ mediated by PEA 80mesh at different concentrations (300-600-1200 mg) compared with Normast® (300-600-1200 mg) after 24h of treatment
- Evaluation of NRG1 and MPZ at increasing concentrations of *Equisetum* (150-300) titrated at 10% in silica
- Evaluation of NRG1 and MPZ at increasing concentrations of PEA 80mesh (300-600-1200 mg) combined with *Equisetum* (150-300mg) titrated at 10% in silica

PEA 80 mesh and Normast® increase the activity of both NRG1 and MPZ indicating that they stimulate myelin synthesis and stabilization inversely proportional to PEA concentration. EquiPEA™ at 600mg+300mg amplifies the effect and demonstrates greater activity on both markers than Normast® ($p < 0.05$) or PEA 80 mesh alone ($p < 0.05$).

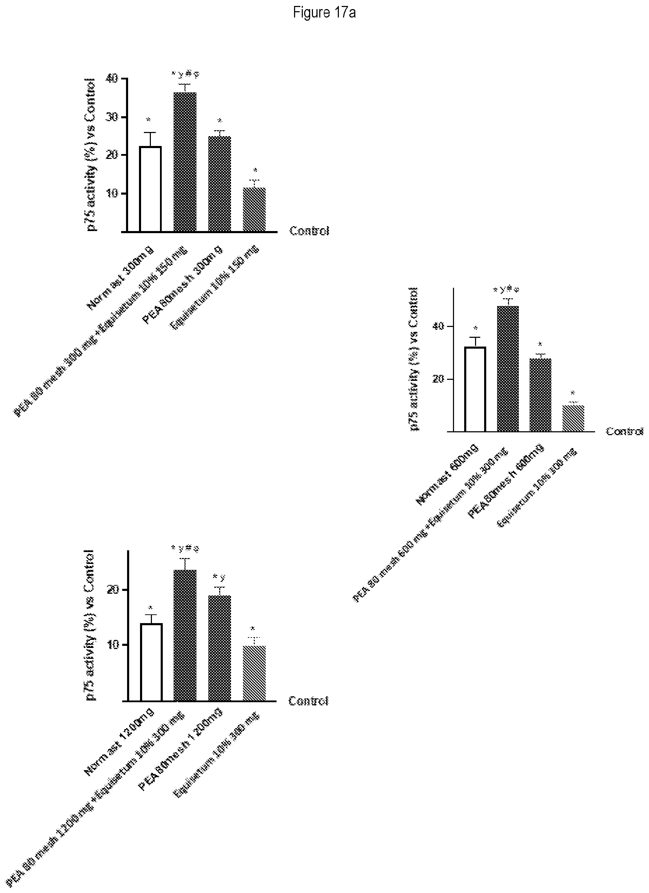

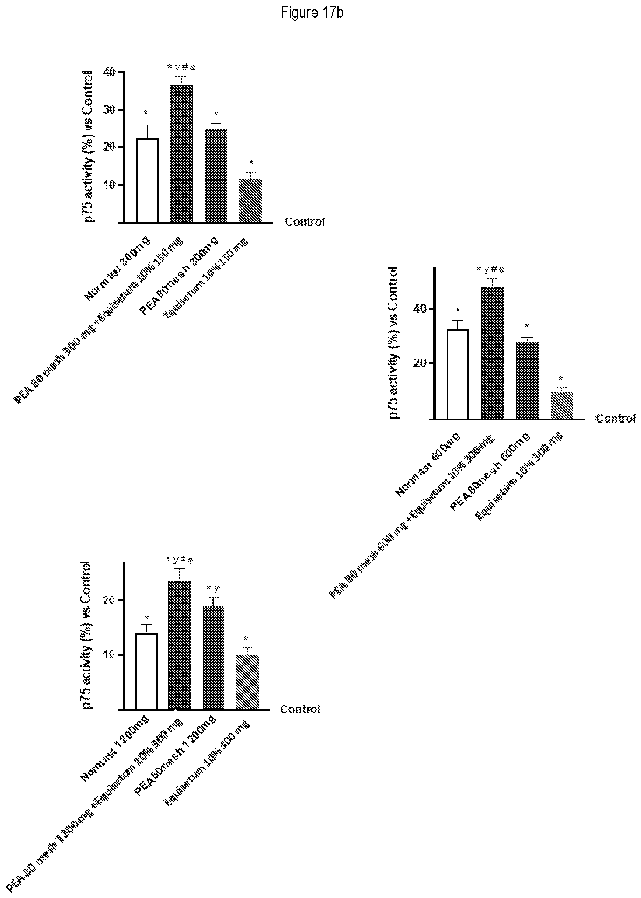

MARKERS (Figure 17a and 17b)

Evaluation of p75 and ERB3 activity to determine treatment functionality (Gongalves NP, et al. 2020. doi: 10.1002/glia.23881; Mizobuchi et al. 2013 doi: 10.2147/JPR.S40967)

Tests conducted:

- Analysis of p75 and ERB3 mediated by PEA 80mesh at different concentrations (300-600-1200 mg) compared with Normast® (300-600-1200 mg) after 24h of treatment
- Evaluation of p75 and ERB3 at increasing concentrations of *Equisetum* (150-300) titrated at 10% in silica
- Evaluation of p75 and ERB3 at increasing concentrations of PEA 80mesh (300-600-1200 mg) combined with *Equisetum* (150-300mg) titrated at 10% in silica

Again, PEA 80 mesh and Normast® increase the activity of the markers, indicating that they regulate myelin synthesis and stabilization supporting use under conditions of peripheral nerve damage. EquiPEA™ at 600mg+300mg amplifies the effect and shows to be more effective on both markers than Normast® ($p < 0.05$) or PEA 80 mesh alone ($p < 0.05$)

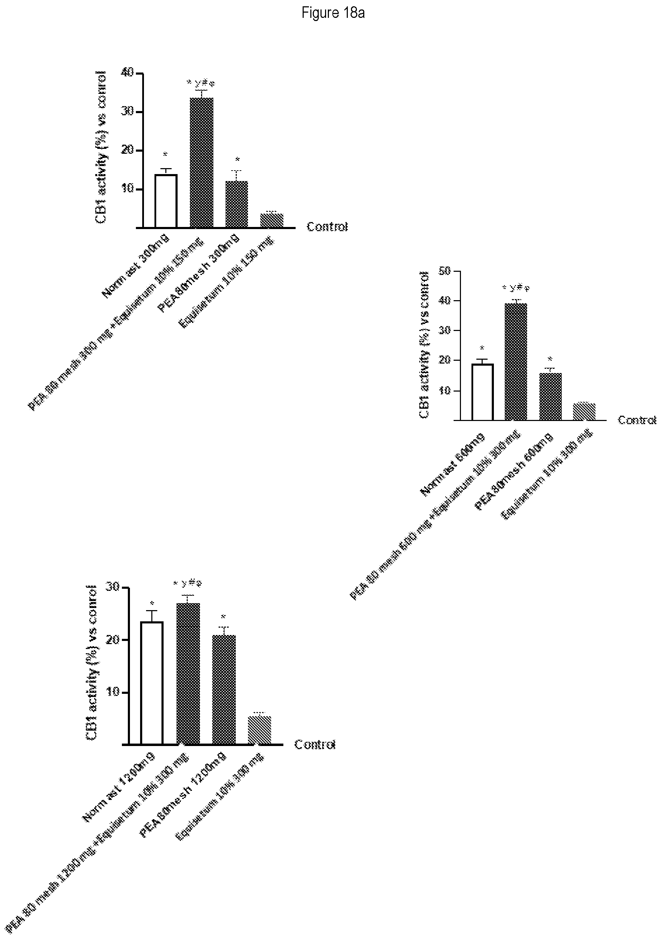

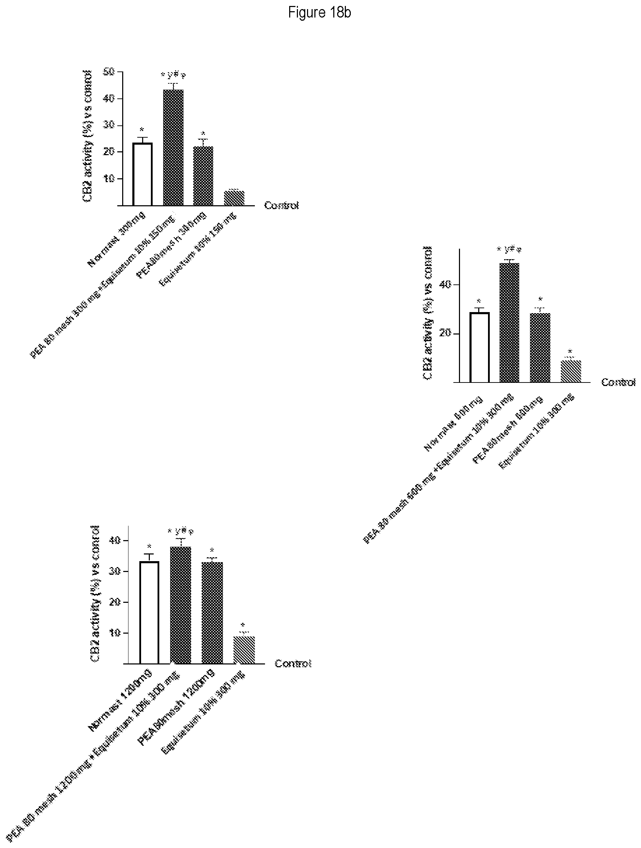

MARKERS (Figure 18a and 18b)

Evaluation of endocannabinoid receptor (CB1 and CB2) activity (McKenna et al. 2020. doi: 1 0.1111/bph.15208)

Tests conducted:

- Analysis of CB1 and CB2 mediated by PEA 80mesh at different concentrations (300-600-1200 mg) compared with Normast® (300-600-1200 mg) after 24h of treatment
- Evaluation of CB1 and CB2 at increasing concentrations of *Equisetum* (150-300) titrated at 10% in silica
- Evaluation of CB1 and CB2 at increasing concentrations of PEA 80mesh (300-600-1200 mg) combined with *Equisetum* (150-300mg) titrated at 10% in silica

The aim was to verify that the main mechanisms of PEA were kept active, thus improving its functionality. EquiPEA™ at 600mg+300mg amplifies the effect and shows to be more effective on both receptors than Normast® ($p < 0.05$) or 80 mesh PEA alone ($p < 0.05$). The activity on endocannabinoid receptors is of interest in understanding the function of axons in transmission and of pain nociceptors.

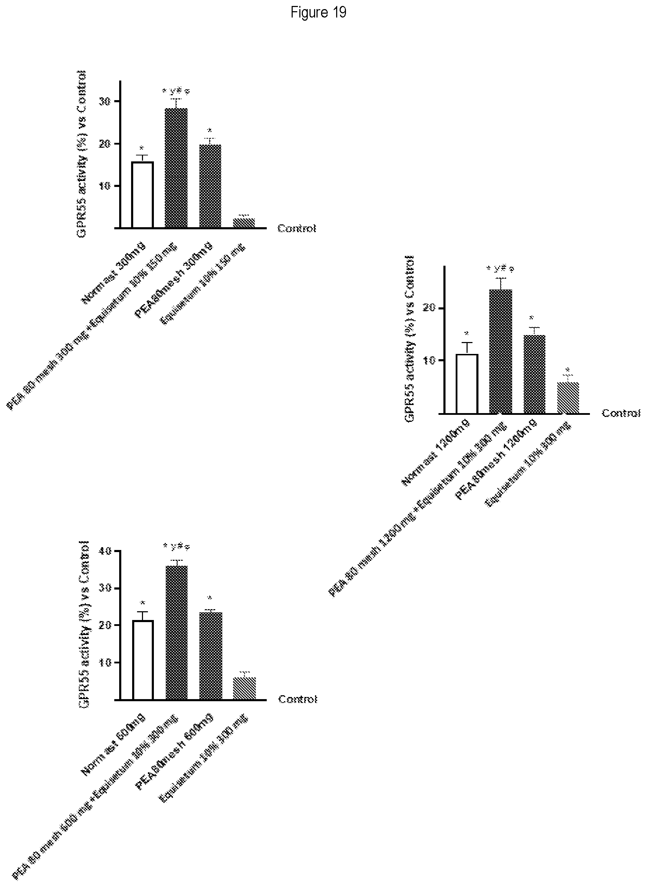

<u>MARKERS</u> (Figure 19)

<u>Evaluation of G-protein-coupled receptor 55 (GPR55) activity</u> (Armin et al. 2021. doi: 10.1016/j.bbr.2021.113248)

Tests conducted:

- Analysis of PEA 80mesh-mediated GPR55 at different concentrations (300-600-1200 mg) compared with Normast® (300-600-1200 mg) after 24h of treatment
- Evaluation of GPR55 at increasing concentrations of *Equisetum* (150-300) titrated at 10% in silica
- Evaluation of GPR55 at increasing concentrations of PEA 80mesh (300-600-1200 mg) combined with *Equisetum* (150-300mg) titrated at 10% in silica

Since the main goal is pain reduction the "third" endocannabinoid receptor was analyzed because it is more responsible for such modulation. EquiPEA™ at 600mg+300mg amplifies the effect and is shown to be more effective on the receptor than Normast® ($p<0.05$) or 80 mesh PEA alone ($p<0.05$), being a calcium uptake marker correlated with gabaergic activity.

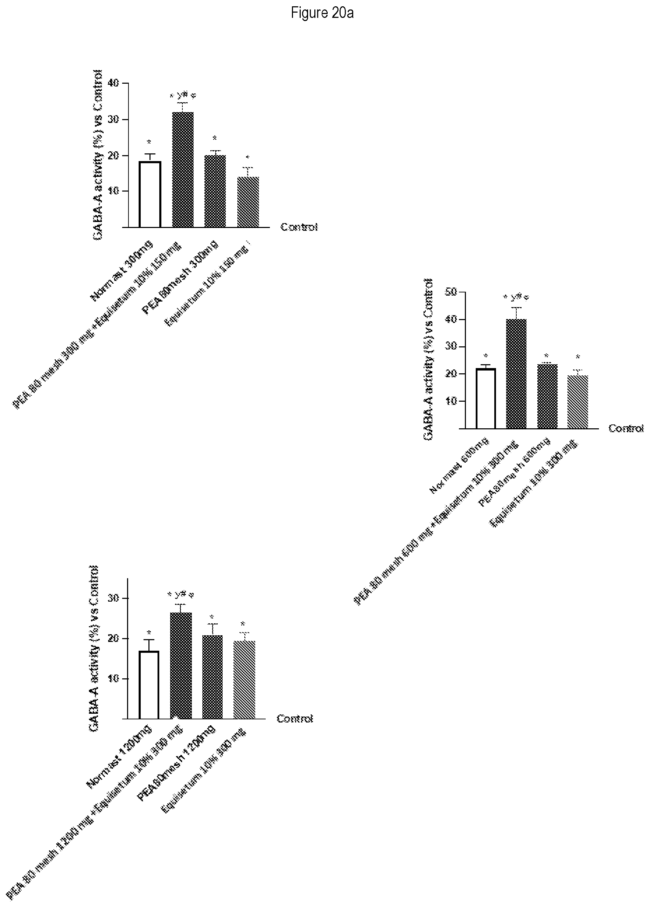

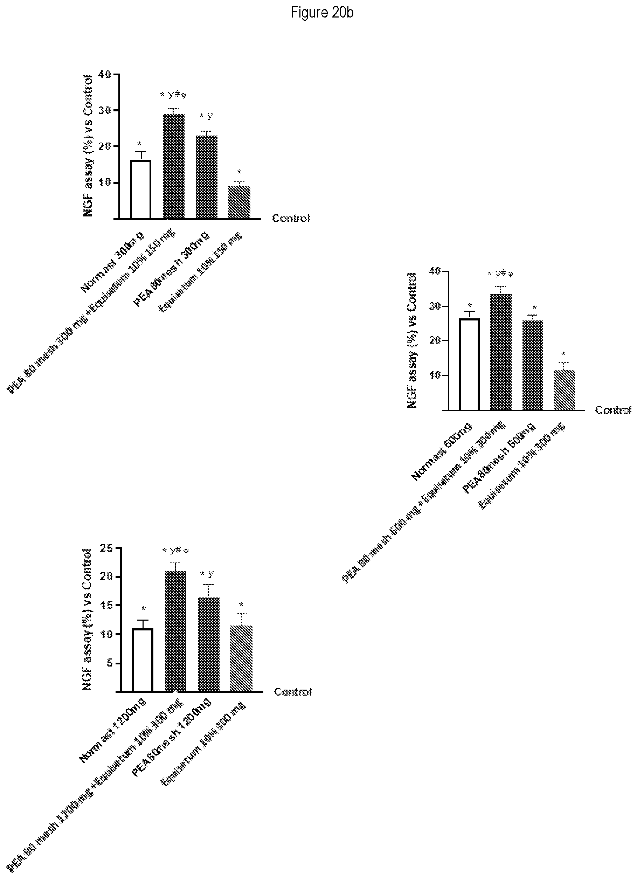

<u>MARKERS</u> (Figure 20a and 20b)

<u>Evaluation of GABA activity and NGF production</u> (Serrano-Regal et al. 2020. doi: 10.3389/fncel.2020.00256.; Li et al. 2020. doi: 10.1038/s41401-019-0338-1)

Tests conducted:

- Analysis of GABA and NGF mediated by PEA 80mesh at different concentrations (300-600-1200 mg) compared with Normast® (300-600-1200 mg) after 24h of treatment
- Evaluation of GABA and NGF at increasing concentrations of *Equisetum* (150-300) titrated at 10% in silica
- Evaluation of GABA and NGF at increasing concentrations of PEA 80mesh (300-600-1200 mg) combined with *Equisetum* (150-300mg) titrated at 10% in silica

The positive role on myelination is confirmed: in particular, it can be observed that EquiPEA™ at 600mg+300mg amplifies the effect, demonstrating the active role of *Equisetum* on GABA compared to Normast® ($p<0.05$) or PEA 80 mesh alone ($p<0.05$). In addition, the promotion of NGF factor, stimulates post-oxidative trauma neuronal repair more effectively with EquiPEA™ at 600mg+300mg than the other forms tested ($p<0.05$).

**Claims**

1. A food, nutraceutical, and/or pharmaceutical combination that comprises or, alternatively, consists of a palmitoylethanolamide (PEA) and an *Equisetum.*

2. The combination according to claim 1, **characterized by** the fact that said *Equisetum* is *Equisetum Arvense.*

3. The combination according to claim 1 or 2, **characterized by** the fact that the palmitoylethanolamide/Equisetum weight ratio is from 1/1 to 10/1, preferably from 2/1 to 4/1; said ratio being based considering *Equisetum* in powder form titrated in silica at a percentage from 1% to 20%, preferably from 5% to 15%, even more preferably from 7% to 10% in silica.

4. The combination according to any one of claims 1 to 3 , wherein said palmitoylethanolamide is palmitoylethanolamide having preferably an average fineness or size or average particle size ranging from 20 mesh to 120 mesh, preferably from 40 mesh to 100 mesh, even more preferably from 60 mesh to 80 mesh.

5. The combination according to any one of claims 1-4, wherein said combination comprises PEA from 100 mg to 1,500 mg, preferably from 300 mg to 1,200 mg, and *Equisetum* from 50 mg to 1,000 mg, preferably from 150 mg to 600 mg, said amount being based considering *Equisetum* in powder form titrated preferably at 10% in silica.

6. The combination according to claim 5, wherein said combination comprises 300 mg of PEA and 150 mg of *Equisetum,* preferably comprises 600 mg of PEA and 300 mg of *Equisetum.*

7. The combination according to any one of claims 1-6, wherein said combination is (i) for use in a method for the neuronal protection and regeneration of the peripheral nervous system and/or (ii) for use in method for the prevention and repair of peripheral nervous system damage.

8. A food, nutraceutical, and/or pharmaceutical composition comprising the combination according to any one of claims 1 to 7, preferably together with one or more food-grade or pharmaceutical grade excipients, and/or physiologically acceptable vehicles; preferably said composition is (i) for use in a method for the neuronal protection and regeneration of the peripheral nervous system and/or (ii) for use in method for the prevention and repair of peripheral nervous system damage.

9. The composition according to claim 8, wherein said composition is suitable for oral administration.

10. A kit comprising (a) at least one food, nutraceutical and/or pharmaceutical composition comprising PEA according to any one of claims 1-7, preferably together with one or more physiologically acceptable excipients or vehicles, and (b) at least one food, nutraceutical and/or pharmaceutical composition comprising *Equisetum* according to any one of claims 1-7, preferably together with one or more physiologically acceptable excipients or vehicles.

**Patentansprüche**

1. Lebensmittel-, nutrazeutische und/oder pharmazeutische Kombination, die ein Palmitoylethanolamid (PEA) und ein Equisetum umfasst oder alternativ daraus besteht.

2. Kombination nach Anspruch 1, **dadurch gekennzeichnet, dass** das Equisetum Equisetum arvense ist.

3. Kombination nach Anspruch 1 oder 2, **dadurch gekennzeichnet, dass** das Gewichtsverhältnis Palmitoylethanolamid/Equisetum von 1/1 bis 10/1, vorzugsweise von 2/1 bis 4/1, beträgt; wobei das Verhältnis bezogen ist auf Equisetum in Pulverform in Siliciumdioxid titriert auf einen Anteil von 1 % bis 20 %, vorzugsweise von 5 % bis 15%, noch bevorzugter von 7 % bis 10 % in Siliciumdioxid.

4. Kombination nach einem der Ansprüche 1 bis 3, wobei das Palmitoylethanolamid Palmitoylethanolamid mit vorzugsweise einer mittleren Feinheit oder Größe oder mittleren Partikelgröße in dem Bereich von 20 Mesh bis 120 Mesh, vorzugsweise von 40 Mesh bis 100 Mesh, noch mehr bevorzugt von 60 Mesh bis 80 Mesh, ist.

5. Kombination nach einem der Ansprüche 1-4, wobei die Kombination PEA von 100 mg bis 1.500 mg, vorzugsweise von 300 mg bis 1.200 mg, und Equisetum von 50 mg bis 1.000 mg, vorzugsweise von 150 mg bis 600 mg, umfasst, wobei die Menge bezogen ist auf Equisetum in Pulverform titriert vorzugsweise auf 10 % in Siliciumdioxid.

6. Kombination nach Anspruch 5, wobei die Kombination 300 mg PEA und 150 mg Equisetum umfasst, vorzugsweise 600 mg PEA und 300 mg Equisetum umfasst.

7. Kombination nach einem der Ansprüche 1-6, wobei die Kombination (i) zur Verwendung bei einem Verfahren zum neuronalen Schutz und zur Regeneration des peripheren Nervensystems und/oder (ii) zur Verwendung bei einem Verfahren zur Prävention und Reparatur von Schäden des peripheren Nervensystems ist.

8. Lebensmittel-, nutrazeutische und/oder pharmazeutische Zusammensetzung umfassend die Kombination nach einem der Ansprüche 1 bis 7, vorzugsweise zusammen mit einem oder mehreren lebensmittelgeeigneten oder pharmazeutisch geeigneten Hilfsstoffen und/oder physiologisch annehmbaren Trägern; wobei die Zusammensetzung (i) vorzugsweise zur Verwendung bei einem Verfahren zum neuronalen Schutz und zur Regeneration des peripheren Nervensystems und/oder (ii) zur Verwendung bei einem Verfahren zur Prävention und Reparatur von Schäden des peripheren Nervensystems ist.

9. Zusammensetzung nach Anspruch 8, wobei die Zusammensetzung zur oralen Verabreichung geeignet ist.

**10.** Kit, umfassend (a) wenigstens eine Lebensmittel-, nutrazeutische und/oder pharmazeutische Zusammensetzung, die PEA umfasst, nach einem der Ansprüche 1-7, vorzugsweise zusammen mit einem oder mehreren physiologisch annehmbaren Hilfsstoffen oder Trägern, und (b) wenigstens eine Lebensmittel-, nutrazeutische und/oder pharmazeutische Zusammensetzung, die Equisetum umfasst, nach einem der Ansprüche 1-7, vorzugsweise zusammen mit einem oder mehreren physiologisch annehmbaren Hilfsstoffen oder Trägern.

## Revendications

**1.** Combinaison alimentaire, nutraceutique et/ou pharmaceutique qui comprend un palmitoyléthanolamide (PEA) et un *Equisetum* ou, en variante, qui est constituée de ceux-ci.

**2.** Combinaison selon la revendication 1, **caractérisée par le fait que** ledit *Equisetum* est *Equisetum arvense.*

**3.** Combinaison selon la revendication 1 ou 2, **caractérisée par le fait que** le rapport pondéral *palmitoyléthanolamide*/*Equisetum* est de 1/1 à 10/1, de préférence de 2/1 à 4/1 ; ledit rapport étant basé sur la prise en compte *d'Equisetum* sous forme de poudre titrée en silice à un pourcentage allant de 1 % à 20 %, de préférence de 5 % à 15 %, encore plus préférablement de 7 % à 10 % en silice.

**4.** Combinaison selon l'une quelconque des revendications 1 à 3, dans laquelle ledit palmitoyléthanolamide est du palmitoyléthanolamide ayant de préférence une finesse ou taille moyenne ou une taille moyenne des particules allant de 20 mesh à 120 mesh, de préférence de 40 mesh à 100 mesh, encore plus préférablement de 60 mesh à 80 mesh.

**5.** Combinaison selon l'une quelconque des revendications 1 à 4, ladite combinaison comprenant du PEA à hauteur de 100 mg à 1 500 mg, de préférence de 300 mg à 1 200 mg, et de *l'Equisetum* à hauteur de 50 mg à 1 000 mg, de préférence de 150 mg à 600 mg, ladite quantité étant basée sur la prise en compte *d'Equisetum* sous forme de poudre titrée de préférence à 10 % en silice.

**6.** Combinaison selon la revendication 5, ladite combinaison comprenant 300 mg de PEA et 150 mg *d'Equisetum,* de préférence 600 mg de PEA et 300 mg *d'Equisetum.*

**7.** Combinaison selon l'une quelconque des revendications 1 à 6, ladite combinaison étant (i) destinée à être utilisée dans une méthode pour la protection et la régénération neuronales du système nerveux périphérique et/ou (ii) destinée à être utilisée dans une méthode pour la prévention et la réparation de lésions du système nerveux périphérique.

**8.** Composition alimentaire, nutraceutique et/ou pharmaceutique comprenant la combinaison selon l'une quelconque des revendications 1 à 7, de préférence conjointement avec un ou plusieurs excipients de qualité alimentaire ou de qualité pharmaceutique et/ou véhicules physiologiquement acceptables ; de préférence ladite composition étant (i) destinée à être utilisée dans une méthode pour la protection et la régénération neuronales du système nerveux périphérique et/ou (ii) destinée à être utilisée dans une méthode pour la prévention et la réparation de lésions du système nerveux périphérique.

**9.** Composition selon la revendication 8, ladite composition étant appropriée pour une administration orale.

**10.** Kit comprenant (a) au moins une composition alimentaire, nutraceutique et/ou pharmaceutique comprenant du PEA selon l'une quelconque des revendications 1 à 7, de préférence conjointement avec un ou plusieurs excipients ou véhicules physiologiquement acceptables, et (b) au moins une composition alimentaire, nutraceutique et/ou pharmaceutique comprenant de *l'Equisetum* selon l'une quelconque des revendications 1 à 7, de préférence conjointement avec un ou plusieurs excipients ou véhicules physiologiquement acceptables.

Figure 1

Figure 2

Figure 3

Figure 4

Figure 5

Figure 6

EP 4 565 075 B1

Figure 7

Figure 8

Figure 9

Figure 10

Figure 11

Figure 12

Figure 13

Figure 14

Figure 15°

EP 4 565 075 B1

Figure 15b

Figure 16a

Figure 16b

Figure 17a

Figure 17b

Figure 18a

Figure 18b

Figure 19

Figure 20a

Figure 20b

**EP 4 565 075 B1**

**Non-patent literature cited in the description**

- **MORSANUTO V et al.** *TEER and Papp analysis*, 2020